# EUROPEAN PATENT APPLICATION

(11) **EP 4 501 966 A1**
(43) Date of publication of application: **05.02.2025**
(21) Application number: 23824301.8
(22) Date of filing: 16.06.2023
(51) Int. Cl.: C07K 16/28

(54) **ANTIBODY SPECIFICALLY BINDING TO HUMAN DR3 AND USE THEREOF**

(30) Priority: 17.06.2022 KR 20220074078; 15.06.2023 KR 20230077023
(71) Applicant: Selecxine Inc., Pohang-si, Gyeongsangbuk-do 37666 (KR)
(72) Inventor: MAENG, Jiyoung, Cheonan-si, Chungcheongnam-do 31174 (KR); KIM, Daeun, Seoul 05837 (KR); KIM, Geona, Seoul 05547 (KR); SHIN, Hyewon, Seoul 05825 (KR); LEE, Jun-Young, Seoul 05854 (KR)
(74) Representative: Dehns
(86) International application number: PCT/KR2023/008410
(87) International publication number: WO 2023/244089

(57) **Abstract**

The present invention relates to an antibody specifically binding to human DR3 and a use thereof, which can specifically bind to a specific epitope of DR3 and act selectively on regulatory T cells, thereby strongly activating only the regulatory T cells while minimizing the activity of CD4⁺ T cells and CD8⁺ T cells that show effector activity. By inducing such selective activity for regulatory T cells, the antibody has an excellent effect of preventing or treating graft-versus-host disease, autoimmune diseases, and type 2 diabetes, and thus can be effectively used as a therapeutic agent for various diseases related thereto.

## Description

### [Technical Field]

The present invention relates to an antibody specifically binding to human DR3 and a use thereof.

The present application claims priority to and the benefit of Korean Patent Application Nos. 10-2022-0074078 and 10-2023-0077023 filed in the Korean Intellectual Property Office on June 17, 2022, and June 15, 2023, respectively, and all the contents disclosed in the specification and drawings of these applications are incorporated in the present application.

### [Background Art]

Death receptor 3 (DR3, tumor necrosis factor receptor superfamily 25, TNFRSF 25) is one of the TNFRSF members. DR3 consists of 417 amino acids, has a molecular weight of 47 kDa, and is composed of a homotrimer. Although DR3 is highly expressed in regulatory T cells (Treg) in a normal state, the expression of DR3 also increases when conventional T cells are activated. In addition, DR3 is known to be expressed in immune cells such as NK cells, ILCs, B cells, monocytes, and non-immune cells such as osteocytes and endothelial cell colony-forming cells.

Tumor necrosis factor-like ligand 1A (TL1A), which is a ligand of DR3, is mainly expressed in activated T cells, dendritic cells, macrophages, monocytes, and endothelial cells. TL1A activates NF-κB signaling by binding to DR3, thereby functioning as a costimulation factor for T cells. According to a previously reported paper, selective activation of regulatory T cells can be induced using a DR3 agonist. In this case, the DR3 agonist is one of the methods of activating regulatory T cells, and selectively activates only regulatory T cells, unlike IL-2, which activates effector T cells in addition to regulatory T cells. The activation of effector T cells may be a major problem that hinders treatment when treating various inflammatory diseases.

The DR3 agonist may be TL1A which is a ligand for DR3, or an agonistic anti-DR3 antibody. However, since TL1A has a decoy receptor (DcR3), which is a natural inhibitor, and natural inhibitors of cytokines are usually regulated by negative feedback, a method of inducing DR3 activation using TL1A makes it difficult to efficiently deliver a drug, and the characteristics of TL1A as a homotrimeric cytokine may increase the difficulty of the new drug development process. On the other hand, using an agonistic anti-DR3 antibody, negative feedback by DcR3 may be avoided and efficient DR3 activation may be induced.

Meanwhile, research on the clinical applicability of antibody drugs has been continuously conducted since the development of the first monoclonal antibody in 1975, and the mechanism of action through which a specific biological reaction can be very strongly and selectively controlled using the characteristics of antibodies that have strong binding affinity and high binding specificity for antigens is the fundamental core of the potential for developing antibody drugs. In particular, compared to existing therapeutic agents based on chemical synthetic substances, antibody drugs are shown to have relatively fewer side effects and excellent therapeutic efficacy due to their high binding specificity and stability in the human body, and as a result, the field of therapeutic agent development using antibodies is drawing attention as a next-generation core field for new drug research and development.

However, an antibody drug which specifically binds to DR3 to activate regulatory T cells and has excellent therapeutic effects on graft-versus-host disease, autoimmune diseases, or type 2 diabetes has not yet been developed.

### [Disclosure]

### [Technical Problem]

The present invention is directed to providing an anti-DR3 antibody or an antigen-binding fragment thereof, which specifically binds to death receptor 3 (DR3).

The present invention is also directed to providing a nucleic acid sequence encoding the antibody or antigen-binding fragment.

The present invention is also directed to providing a vector including the nucleic acid sequence.

The present invention is also directed to providing a transgenic organism transformed with the vector.

The present invention is also directed to providing a pharmaceutical composition for preventing or treating graft-versus-host disease, autoimmune diseases, or type 2 diabetes, including, as an active ingredient, the anti-DR3 antibody or the antigen-binding fragment thereof, which specifically binds to death receptor 3 (DR3).

The present invention is also directed to providing a kit for preventing or treating graft-versus-host disease, autoimmune diseases, or type 2 diabetes, including the vector or the composition, and instructions.

However, the technical problems which the present invention intends to solve are not limited to the technical problems that have been mentioned above, and other technical problems, which have not been mentioned, will be clearly understood by a person with ordinary skill in the art to which the present invention pertains from the following description.

### [Technical Solution]

One aspect of the present invention provides an anti-DR3 antibody or an antigen-binding fragment thereof, which specifically binds to death receptor 3 (DR3).

Another aspect of the present invention provides a nucleic acid sequence encoding the antibody or antigen-binding fragment.

Still another aspect of the present invention provides a vector including the nucleic acid sequence.

Yet another aspect of the present invention provides a transgenic organism transformed with the vector.

Yet another aspect of the present invention provides a pharmaceutical composition for preventing or treating graft-versus-host disease, autoimmune diseases, or type 2 diabetes, including, as an active ingredient, the anti-DR3 antibody or the antigen-binding fragment thereof, which specifically binds to death receptor 3 (DR3).

Yet another aspect of the present invention provides a kit for preventing or treating graft-versus-host disease, autoimmune diseases, or type 2 diabetes, including the vector or the composition, and instructions.

In an exemplary embodiment of the present invention, the antibody or the antigen-binding fragment may be any one selected from the group consisting of the following, but is not limited thereto:
i) an antibody or antigen-binding fragment including a heavy chain variable region including a heavy chain CDR1 set forth in an amino acid sequence of SEQ ID NO: 1, a heavy chain CDR2 set forth in an amino acid sequence of SEQ ID NO: 2, and a heavy chain CDR3 set forth in an amino acid sequence of SEQ ID NO: 3; and a light chain variable region including a light chain CDR1 set forth in an amino acid sequence of SEQ ID NO: 4, a light chain CDR2 set forth in an amino acid sequence of SEQ ID NO: 5, and a light chain CDR3 set forth in an amino acid sequence of SEQ ID NO: 6;
ii) an antibody or antigen-binding fragment including a heavy chain variable region including a heavy chain CDR1 set forth in an amino acid sequence of SEQ ID NO: 7, a heavy chain CDR2 set forth in an amino acid sequence of SEQ ID NO: 8, and a heavy chain CDR3 set forth in an amino acid sequence of SEQ ID NO: 9; and a light chain variable region including a light chain CDR1 set forth in an amino acid sequence of SEQ ID NO: 10, a light chain CDR2 set forth in an amino acid sequence of SEQ ID NO: 11, and a light chain CDR3 set forth in an amino acid sequence of SEQ ID NO: 12;
iii) an antibody or antigen-binding fragment including a heavy chain variable region including a heavy chain CDR1 set forth in an amino acid sequence of SEQ ID NO: 13, a heavy chain CDR2 set forth in an amino acid sequence of SEQ ID NO: 14, and a heavy chain CDR3 set forth in an amino acid sequence of SEQ ID NO: 15; and a light chain variable region including a light chain CDR1 set forth in an amino acid sequence of SEQ ID NO: 16, a light chain CDR2 set forth in an amino acid sequence of SEQ ID NO: 17, and a light chain CDR3 set forth in an amino acid sequence of SEQ ID NO: 18;
iv) an antibody or antigen-binding fragment including a heavy chain variable region including a heavy chain CDR1 set forth in an amino acid sequence of SEQ ID NO: 19, a heavy chain CDR2 set forth in an amino acid sequence of SEQ ID NO: 20, and a heavy chain CDR3 set forth in an amino acid sequence of SEQ ID NO: 21; and a light chain variable region including a light chain CDR1 set forth in an amino acid sequence of SEQ ID NO: 22, a light chain CDR2 set forth in an amino acid sequence of SEQ ID NO: 23, and a light chain CDR3 set forth in an amino acid sequence of SEQ ID NO: 24; and
v) an antibody or antigen-binding fragment including a heavy chain variable region including a heavy chain CDR1 set forth in an amino acid sequence of SEQ ID NO: 25, a heavy chain CDR2 set forth in an amino acid sequence of SEQ ID NO: 26, and a heavy chain CDR3 set forth in an amino acid sequence of SEQ ID NO: 27; and a light chain variable region including a light chain CDR1 set forth in an amino acid sequence of SEQ ID NO: 28, a light chain CDR2 set forth in an amino acid sequence of SEQ ID NO: 29, and a light chain CDR3 set forth in an amino acid sequence of SEQ ID NO: 30.

In an exemplary embodiment of the present invention, the antibody or the antigen-binding fragment may be any one selected from the group consisting of the following, but is not limited thereto:
i) an antibody or antigen-binding fragment thereof including a heavy chain variable region set forth in an amino acid sequence of SEQ ID NO: 63 and a light chain variable region set forth in an amino acid sequence of SEQ ID NO: 64;
ii) an antibody or antigen-binding fragment thereof including a heavy chain variable region set forth in an amino acid sequence of SEQ ID NO: 67 and a light chain variable region set forth in an amino acid sequence of SEQ ID NO: 68;
iii) an antibody or antigen-binding fragment thereof including a heavy chain variable region set forth in an amino acid sequence of SEQ ID NO: 71 and a light chain variable region set forth in an amino acid sequence of SEQ ID NO: 72;
iv) an antibody or antigen-binding fragment thereof including a heavy chain variable region set forth in an amino acid sequence of SEQ ID NO: 75 and a light chain variable region set forth in an amino acid sequence of SEQ ID NO: 76; and
v) an antibody or antigen-binding fragment thereof including a heavy chain variable region set forth in an amino acid sequence of SEQ ID NO: 79 and a light chain variable region set forth in an amino acid sequence of SEQ ID NO: 80.

In an exemplary embodiment of the present invention, the antibody or the antigen-binding fragment may include a heavy chain set forth in an amino acid sequence of SEQ ID NO: 83 and a light chain set forth in an amino acid sequence of SEQ ID NO: 84, but is not limited thereto.

In an exemplary embodiment of the present invention, the antibody or the antigen-binding fragment may include Fab, but is not limited thereto.

In an exemplary embodiment of the present invention, the antibody or the antigen-binding fragment may be IgG1, or IgG4 subclass, but is not limited thereto.

In an exemplary embodiment of the present invention, the antigen-binding fragment may be any one selected from the group consisting of Fv, Fab, F(ab')2, Fab', dsFv, scFv, sc(Fv)2, and a diabody, but is not limited thereto.

In an exemplary embodiment of the present invention, the iii), iv), and v) antibody or antigen-binding fragment may specifically bind to DR3 competitively with TL1A, but is not limited thereto.

In an exemplary embodiment of the present invention, the antibody or the antigen-binding fragment may be characterized by any one or more of the following, but is not limited to:
selectively activating regulatory T cells or enhancing the survival rate thereof; and
activating NF-κB.

In an exemplary embodiment of the present invention, the autoimmune disease may be any one or more selected from the group consisting of an autoimmune disease caused by graft-versus-host disease, hemophagocytic lymphohistiocytosis, systemic lupus erythematosus, Kikuchi disease, adult onset Stills disease, Behcet disease, IgG4-related disease, type 1 diabetes, systemic sclerosis, psoriasis, multiple sclerosis, and Graves hyperthyroidism, but is not limited thereto.

Yet another aspect of the present invention provides a method for preventing or treating graft-versus-host disease, autoimmune diseases, or type 2 diabetes, the method including administering an anti-DR3 antibody or an antigen-binding fragment thereof, which binds to death receptor 3 (DR3), or a composition including the same as an active ingredient to a subject in need thereof.

Yet another aspect of the present invention provides a use of an anti-DR3 antibody or an antigen-binding fragment thereof, which binds to death receptor 3 (DR3), or a composition including the same as an active ingredient for preventing or treating graft-versus-host disease, autoimmune diseases, or type 2 diabetes.

Yet another aspect of the present invention provides a use of an anti-DR3 antibody or an antigen-binding fragment thereof, which binds to death receptor 3 (DR3), or a composition including the same as an active ingredient for preparing a preparation for preventing or treating graft-versus-host disease, autoimmune diseases, or type 2 diabetes.

### [Advantageous Effects]

An antibody which specifically binds to human DR3 and a use thereof can improve the division or survival rate of regulatory T cells by specifically binding to a specific epitope of human DR3 and selectively acting on regulatory T cells while minimizing the activity of effector CD4+ T cells and CD8+ T cells. Since the antibody has a remarkably excellent preventive or therapeutic effect against related diseases due to such activity, the antibody can be usefully used as a therapeutic agent for graft-versus-host disease, autoimmune diseases, or type 2 diabetes.

### [Description of Drawings]

FIGS. 1A and 1B show the experimental results of a process of discovering an anti-DR3 scFv clone which specifically binds to DR3 through phage display.
FIGS. 2A to 2C show the binding specificity and affinity of anti-DR3 monoclonal antibodies to DR3.
FIGS. 3A and 3B show the results of confirming that the binding site of an anti-DR34-4 antibody for DR3 overlaps with the binding site of TL1A.
FIG 4 shows the NF-κB activation level of the anti-DR3 antibody of the present invention.
FIG 5 shows the results of comparing the affinity of anti-DR3 4-4 antibodies to DR3 according to IgG subclass.
FIG 6 shows the results of a comparative analysis of the NF-κB activation levels of anti-DR3 4-4 antibodies according to IgG subclass.
FIG 7 shows the effect of anti-DR3 4-4 IgG4 antibody on increasing the survival rate of regulatory T cells in the absence of TCR signal stimulation.
FIG 8 shows the effect of anti-DR3 4-4 IgG4 antibody on regulating the division and survival rate of regulatory T cells according to TCR signal intensity.

### [Best Mods]

In the present invention, an anti-DR3 antibody which binds to the DR3 natural form was discovered using fully human phage display and a stable cell line expressing DR3. It was confirmed that the anti-DR3 antibody of the present invention can function as an agonist by activating T cells through competitive binding with TL1A to DR3 expressed by human T cells. Further, the activation degrees of T cells by each isotype of an antibody were compared by preparing antibodies for clone 4-4 which activates T cells most strongly among them to have different isotypes such as IgG1, IgG2, and IgG4, and it was confirmed that the anti-DR3 IgG4 antibody specifically binds to the DR3 natural form and activates T cells most strongly. In addition, through this, it was confirmed that the anti-DR3 4-4 IgG4 antibody, which was identified as the most excellent antibody, increases the survival rate of regulatory T cells in the absence of TCR signal stimulation, and effectively increases the division or survival rate of regulatory T cells in the presence of TCR signal stimulation of a certain intensity or higher, and thereby the present invention was completed.

The present invention provides an anti-DR3 antibody or an antigen-binding fragment thereof, which specifically binds to death receptor 3 (DR3).

In the present invention, DR3 (TNFRSF25, TNF receptor superfamily member 25, death receptor 3) is a member of the tumor necrosis factor receptor superfamily which is expressed at high levels in regulatory T cells but may also be induced in existing T cells upon TCR stimulation. In the present invention, DR3 may include DR3, hDR3, and the like, but is not limited thereto. DR3 signaling may promote the activation and proliferation of regulatory T cells in a normal state, as demonstrated in mice with agonist anti-DR3 antibodies, and only small amounts of DR3 may induce the proliferation of regulatory T cells without proliferation of non-target immune cell populations, which is a key difference from an IL-2 complex, which also promotes the division of CD4 and CD8 T cells. Therefore, since the division of conventional T cells (Tconv) may be a major obstacle to the treatment of various inflammatory diseases, anti-DR3 antibodies may be used as a new method of selectively activating regulatory T cells.

In an exemplary embodiment of the present invention, the antibody or the antigen-binding fragment may be any one selected from the group consisting of the following, but is not limited thereto:
i) an antibody or antigen-binding fragment including a heavy chain variable region including a heavy chain CDR1 set forth in an amino acid sequence of SEQ ID NO: 1, a heavy chain CDR2 set forth in an amino acid sequence of SEQ ID NO: 2, and a heavy chain CDR3 set forth in an amino acid sequence of SEQ ID NO: 3; and a light chain variable region including a light chain CDR1 set forth in an amino acid sequence of SEQ ID NO: 4, a light chain CDR2 set forth in an amino acid sequence of SEQ ID NO: 5, and a light chain CDR3 set forth in an amino acid sequence of SEQ ID NO: 6;
ii) an antibody or antigen-binding fragment including a heavy chain variable region including a heavy chain CDR1 set forth in an amino acid sequence of SEQ ID NO: 7, a heavy chain CDR2 set forth in an amino acid sequence of SEQ ID NO: 8, and a heavy chain CDR3 set forth in an amino acid sequence of SEQ ID NO: 9; and a light chain variable region including a light chain CDR1 set forth in an amino acid sequence of SEQ ID NO: 10, a light chain CDR2 set forth in an amino acid sequence of SEQ ID NO: 11, and a light chain CDR3 set forth in an amino acid sequence of SEQ ID NO: 12;
iii) an antibody or antigen-binding fragment including a heavy chain variable region including a heavy chain CDR1 set forth in an amino acid sequence of SEQ ID NO: 13, a heavy chain CDR2 set forth in an amino acid sequence of SEQ ID NO: 14, and a heavy chain CDR3 set forth in an amino acid sequence of SEQ ID NO: 15; and a light chain variable region including a light chain CDR1 set forth in an amino acid sequence of SEQ ID NO: 16, a light chain CDR2 set forth in an amino acid sequence of SEQ ID NO: 17, and a light chain CDR3 set forth in an amino acid sequence of SEQ ID NO: 18;
iv) an antibody or antigen-binding fragment including a heavy chain variable region including a heavy chain CDR1 set forth in an amino acid sequence of SEQ ID NO: 19, a heavy chain CDR2 set forth in an amino acid sequence of SEQ ID NO: 20, and a heavy chain CDR3 set forth in an amino acid sequence of SEQ ID NO: 21; and a light chain variable region including a light chain CDR1 set forth in an amino acid sequence of SEQ ID NO: 22, a light chain CDR2 set forth in an amino acid sequence of SEQ ID NO: 23, and a light chain CDR3 set forth in an amino acid sequence of SEQ ID NO: 24; and
v) an antibody or antigen-binding fragment including a heavy chain variable region including a heavy chain CDR1 set forth in an amino acid sequence of SEQ ID NO: 25, a heavy chain CDR2 set forth in an amino acid sequence of SEQ ID NO: 26, and a heavy chain CDR3 set forth in an amino acid sequence of SEQ ID NO: 27; and a light chain variable region including a light chain CDR1 set forth in an amino acid sequence of SEQ ID NO: 28, a light chain CDR2 set forth in an amino acid sequence of SEQ ID NO: 29, and a light chain CDR3 set forth in an amino acid sequence of SEQ ID NO: 30.

In an exemplary embodiment of the present invention, the antibody or the antigen-binding fragment may be any one selected from the group consisting of the following, but is not limited thereto:
i) an antibody or antigen-binding fragment thereof including a heavy chain variable region set forth in an amino acid sequence of SEQ ID NO: 63 and a light chain variable region set forth in an amino acid sequence of SEQ ID NO: 64;
ii) an antibody or antigen-binding fragment thereof including a heavy chain variable region set forth in an amino acid sequence of SEQ ID NO: 67 and a light chain variable region set forth in an amino acid sequence of SEQ ID NO: 68;
iii) an antibody or antigen-binding fragment thereof including a heavy chain variable region set forth in an amino acid sequence of SEQ ID NO: 71 and a light chain variable region set forth in an amino acid sequence of SEQ ID NO: 72;
iv) an antibody or antigen-binding fragment thereof including a heavy chain variable region set forth in an amino acid sequence of SEQ ID NO: 75 and a light chain variable region set forth in an amino acid sequence of SEQ ID NO: 76; and
v) an antibody or antigen-binding fragment thereof including a heavy chain variable region set forth in an amino acid sequence of SEQ ID NO: 79 and a light chain variable region set forth in an amino acid sequence of SEQ ID NO: 80.

In the present invention, the terms "percent identity," "sequence identity," "percentage similarity," "sequence similarity," "sequence identity percentage," and the like, as used herein in connection with amino acid sequences and/or nucleic acid sequences, may refer to a measure determined by comparing the degree of similarity of two sequences based on the alignment of the sequences, which maximizes the similarity between aligned amino acid residues or nucleotides and is a function of the number of identical or similar residues or nucleotides, the total number of residues or nucleotides, and the presence and length of gaps in the sequence alignment, but are not limited thereto.

A portion of a polynucleotide or polypeptide sequence may include additions or deletions (that is, gaps) compared to a reference sequence (which does not include additions or deletions) for optimal alignment of the two sequences. The percentage is counted by determining the number of positions which an identical nucleobase or amino acid residue indicates in both sequences to calculate the number of matching positions, dividing the number of matching positions by the total number of positions in the comparison window, and multiplying the result by 100 to calculate a sequence identity percentage.

The term "identical" or "percentage identity" in connection with two or more nucleic acid or polypeptide sequences refers to two or more sequences or subsequences that are identical or have a specified percentage of the identical amino acid residue or nucleotide (that is, about 60% identity to the specified region when compared and aligned for maximum correspondence over a comparison window or indicated region, preferably 65%, 70%, 75%, 80%, 85%, 90%, 91%, 92%, 93%, 94%, 95%, 96%, 97%, 98% or even higher identity), as determined using the BLAST or BLAST 2.0 sequence comparison algorithm using the default parameters described below, or by manual alignment and visual inspection (see, for example, the NCBI website http://www.ncbi.nlm.nih.gov/BLAST/, and the like). Such sequences are called "substantially identical" below.

In the present invention, the amino acid sequence or base sequence may include a base sequence having at least 70% sequence identity, more preferably at least 80%, even more preferably at least 90%, and most preferably at least 95% sequence identity with an amino acid sequence or base sequence represented by each sequence number. For example, the amino acid sequence or base sequence may include a sequence having a sequence homology of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%, but the percentage is not limited.

In the present invention, a naturally occurring antibody is a glycoprotein including at least two heavy (H) chains and two light (L) chains linked by interchain disulfide bonds. Each heavy chain consists of a heavy chain variable region (VH) and a heavy chain constant region. The heavy chain constant region consists of three domains, CH1, CH2 and CH3. Each light chain consists of a light chain variable region (abbreviated as VL) and a light chain constant region. The light chain constant region consists of a domain CL.

The VH and VL regions may be further subdivided into regions having high variability referred to as complementarity determining regions (CDRs), separated by more conserved regions referred to as framework regions (FRs). Each VH and VL consists of three CDRs and four FRs arranged from an amino terminus to a carboxy terminus in the following order: FR1, CDR1, FR2, CDR2, FR3, CDR3, FR4. The variable regions of heavy chains and light chains include a binding domain that interacts with an antigen.

In the present invention, an antibody may be derived from various species such as a mouse, a rat, a rabbit, and a guinea pig, but the species is not limited thereto. In addition, the antibody includes a chimeric antibody having an antibody constant region from one species (preferably) with an antigen-binding site from another species. Furthermore, the antibody includes an antibody having an antigen-binding site of the antibody from a non-human species having a constant region of origin and a framework region. The antibody described herein may be a monoclonal antibody, that is, an antibody molecule having a single molecular composition. Systems and immunological manipulations and techniques for preparing and isolating a hybridoma secreting a monoclonal antibody are known in the art.

As used herein, "antigen" refers to a substance that includes a binding site (epitope), and induces and/or produces an immune response against the epitope. The antigen may include a peptide, a protein, a glycoprotein, a polysaccharide, and a lipid, a portion thereof, and a combination thereof, but is not limited thereto. Non-limiting exemplary antigens include tumor antigens or pathogenic antigens. Further, the term "antigen" may refer to a molecule that triggers an immune response. Such an immune response may involve antibody production or activation of specific immunologically active cells, or both. It will be understood by those skilled in the art that any macromolecule, including virtually all proteins or peptides, can be used as an antigen.

As used herein, "binding site" or "epitope" may refer to an antigenic determinant cluster in a molecule, that is, the part of a molecule that is recognized by the immune system (for example, by an antibody). In the present invention, the epitope may include a portion of DR3 that binds to TL1A or a portion of DR3 that binds to an anti-DR3 antibody, but is not limited thereto.

In an exemplary embodiment of the present invention, the antibody or the antigen-binding fragment may include a heavy chain set forth in an amino acid sequence of SEQ ID NO: 83 and a light chain set forth in an amino acid sequence of SEQ ID NO: 84, but is not limited thereto.

As a result of preparing 4-4 IgG4 antibody, which was confirmed to be the most effective in the present invention, for application to graft-versus-host disease, autoimmune diseases, or type 2 diabetes, in an exemplary embodiment of the present invention, the antibody or the antigen-binding fragment may include an Fab, but is not limited thereto.

Therapeutic antibodies are usually immunoglobulin G (IgG), and IgG is divided into four subclasses (IgG1, IgG2, IgG3, and IgG4). Since the subclasses of IgG have different effector functions, it is important to select a suitable IgG subclass depending on the indication of a therapeutic antibody. In this case, it was confirmed that not only an effector function but also binding affinity and an activation function of an antibody to a target antigen differ depending on IgG class. In addition, the strength of binding affinity by IgG subclasses may differ depending on the type of antigen-antibody.

As a result of confirming an ISOTYPE which is functionally the best for the 4-4 antibody which was confirmed to be the most effective in the present invention, in an exemplary embodiment of the present invention, the antibody or the antigen-binding fragment may be an IgG4 subclass, but is not limited thereto.

In an exemplary embodiment of the present invention, the antibody or the antigen-binding fragment may be IgG1 or an IgG4 subclass, but is not limited thereto.

As used herein, "antigen-binding fragment" refers to a fragment of an antibody that retains a specific antigen-binding ability, and refers to one or more fragments of an antibody, including, for example, (i) an Fab fragment, a monovalent fragment consisting of the VL, VH, CL, and CH domains; (ii) an F(ab')2 fragment, a bivalent fragment including two Fab fragments linked by a disulfide bond at the hinge region; (iii) an Fd fragment consisting of the VH and CH domains; (iv) an Fv fragment consisting of the VL and VH domains of a single arm of an antibody; (v) a single domain antibody fragment consisting of a VH domain; (vi) an isolated complementarity determining region (CDR); and (vii) a binding fragment including a combination of two or more isolated CDRs optionally linked by a synthetic linker. There is also a single chain antibody (or single chain Fv (scFv)) formed using a recombinant method to link the two domains of the FV segment, VL and VH, by a synthetic linker.

In an exemplary embodiment of the present invention, the antigen-binding fragment may be any one selected from the group consisting of Fv, Fab, F(ab')2, Fab', dsFv, scFv, sc(Fv)2, and a diabody, but is not limited thereto.

In the present invention, since an anti-DR3 antibody having a binding position which overlaps with TL1A, a ligand that activates human DR3, was discovered through epitope mapping, in order to develop an agonistic anti-DR3 antibody for activating a target antigen human DR3, the antibody or antigen-binding fragment of the present invention may have the same or overlapping epitope binding to DR3 with TL1A, and in this case, the antibody or antigen-binding fragment of the present invention may competitively and specifically bind to DR3 to induce the activity of regulatory T cells, and the like, but is not limited thereto. In addition, when TL1A and an epitope that binds to DR3 do not overlap, the epitope may bind to DR3 together with TL1A to activate DR3 excellently and non-competitively. Therefore, he anti-DR3 antibody of the present invention may exhibit prophylactic or therapeutic effects against graft-versus-host disease, autoimmune diseases, or type 2 diabetes by binding to DR3 competitively and/or non-competitively with TL1A to activate the function of DR3.

In an exemplary embodiment of the present invention, the iii), iv), and v) antibody or antigen-binding fragment may specifically bind to DR3 competitively with TL1A, but is not limited thereto. That is, the binding site (epitope) of the iii), iv), and v) antibody or antigen-binding fragment to DR3 may overlap with the binding site of TL1A for DR3, but is not limited thereto.

In an exemplary embodiment of the present invention, the antibody or the antigen-binding fragment may be characterized by any one or more of the following, but is not limited to:
selectively activating regulatory T cells or enhancing the survival rate thereof; and
activating NF-κB.

In the present invention, the anti-DR3 antibody may exhibit an activation efficiency of about 10% to 80% against CD3+CD4+ NF-κB depending on the concentration, and may exhibit an activation efficiency of about 1 to 60% against CD3+CD8+ NF-κB, but is not limited thereto. In particular, the anti-DR3 4-4 IgG1 antibody of the present invention may increase the activation efficiency of NF-κB cells by inducing a phosphorylation process of up to about 80% and 60% of CD3+CD4+ NF-κB and CD3+CD8+ NF-κB, respectively, depending on the antibody treatment concentration, but is not limited thereto. In the present invention, the anti-DR3 antibody may exhibit an activation efficiency of more than 10 to 100%, more than 10 to 99%, more than 10 to 98%, more than 10 to 97%, more than 10 to 96%, more than 10 to 95%, more than 10 to 94%, more than 10 to 93%, more than 10 to 92%, more than 10 to 91%, more than 10 to 90%, more than 10 to 89%, more than 10 to 88%, more than 10 to 87%, more than 10 to 86%, more than 10 to 85%, more than 10 to 84%, more than 10 to 83%, more than 10 to 82%, or more than 10 to 81% against CD3+CD4+ NF-κB, and the activation efficiency may be preferably more than 10 to 80%, but is not limited thereto. Furthermore, the anti-DR3 antibody may exhibit an activation efficiency of more than 0 to 100%, more than 0 to 95%, more than 0 to 90%, more than 0 to 85%, more than 0 to 80%, more than 0 to 75%, more than 0 to 70%, more than 0 to 69%, more than 0 to 68%, more than 0 to 67%, more than 0 to 66%, more than 0 to 65%, more than 0 to 64%, more than 0 to 63%, more than 0 to 62%, or more than 0 to 61%, and the activation efficiency may be preferably more than 0 to 60%, but is not limited thereto.

In the present invention, the anti-DR3 antibody may induce the strong expansion of regulatory T cells *in vivo.* For example, the number and percentage of regulatory T cells may be increased in lymph nodes and the spleen, and may be selectively exhibited for regulatory T cells, but are not limited thereto.

In the present invention, the anti-DR3 antibody is shown to have a remarkably lower binding dissociation constant for DR3 than that for TL1A, which is a ligand for DR3, and thus may exhibit excellent binding strength. The anti-DR3 antibody of the present invention may exhibit a binding strength of more than about 2-fold to about 10000-fold, more than about 2-fold to about 9800-fold, more than about 2-fold to about 9600-fold, more than about 2-fold to about 9400-fold, more than about 2-fold to about 9200-fold, more than about 4-fold to about 10000-fold, more than about 4-fold to about 9800-fold, more than about 4-fold to about 9600-fold, more than about 4-fold to about 9400-fold, more than about 4-fold to about 9200-fold, more than about 6-fold to about 10000-fold, more than about 6-fold to about 9800-fold, more than about 6-fold to about 9600-fold, more than about 6-fold to about 9400-fold, more than about 6-fold to about 9200-fold, more than about 8-fold to about 10000-fold, more than about 8-fold to about 9800-fold, more than about 8-fold to about 9600-fold, more than about 8-fold to about 9400-fold, more than about 8-fold to about 9200-fold, more than about 10-fold to about 10000-fold, more than about 10-fold to about 9800-fold, more than about 10-fold to about 9600-fold, more than about 10-fold to about 9400-fold, more than about 10-fold to about 9200-fold, more than about 12-fold to about 10000-fold, more than about 12-fold to about 9800-fold, more than about 12-fold to about 9600-fold, and more than about 12-fold to about 9400-fold, and may exhibit a binding strength of preferably more than about 12-fold to about 9200-fold, compared to TL1A, but the binding strength is not limited thereto.

The present invention provides a nucleic acid sequence encoding the antibody or antigen-binding fragment.

As used herein, "genome" may refer to a genetic material (for example, a chromosome) of an organism, and "nucleic acid sequence" or "gene" may refer to a nucleic acid (for example, DNA or RNA) sequence that includes a coding sequence required for the production of a polypeptide or precursor (for example, proinsulin), but is not limited thereto.

"Nucleotide sequence" or "base sequence" in the present invention refers to any nucleotide sequence (for example, RNA or DNA) whose manipulation may also be thought to be preferred for any reason (for example, to treat a disease or improve a property, to express a protein of the present invention in a host cell, or to impart the expression of a ribozyme) by one of ordinary skill in the art. Such nucleotide sequences may include, but are not limited to, coding sequences of a structural gene (for example, a reporter gene, a selectable marker gene, an oncogene, a drug resistance gene, a growth factor, and the like) and non-coding regulatory sequences that do not encode an mRNA or protein product (for example, promoter sequences, polyadenylation sequences, termination sequences, enhancer sequences, and the like).

The present invention provides a vector including the nucleic acid sequence.

"Vector" in the present invention refers to any genetic element, such as a plasmid, phage, transposon, cosmid, chromosome, virus, and virion, which is capable of replicating and transferring a genetic sequence between cells when linked with an appropriate regulatory element.

As used herein, "recombinant expression vector" refers to a bacterial plasmid, a phage, a yeast plasmid, a plant cell virus, a mammalian cell virus or other vectors. In general, any plasmid or vector may be used as long as it can be replicated and stabilized in a host.

The recombinant expression vector and an expression vector including appropriate transcriptional/translational control signals may be constructed by methods well known to those skilled in the art. The method includes *in vitro* recombinant DNA techniques, DNA synthesis techniques, *in vivo* recombination techniques, and the like.

Further, as the vector used for recombination, both a viral vector and a non-viral vector may be used. For example, as the viral vector, a lentiviral, retroviral, adenoviral, herpes viral, or avipox viral vector, and the like may be used, but the viral vector is not limited thereto.

In addition, the vector may further include a tagging gene for increasing the production amount of a recombinant protein, a tagging gene for maintaining the structural stability of the recombinant protein, a tagging gene for easily isolating the recombinant protein, a selectable marker gene such as an antibiotic resistance gene for selecting a transgenic organism, and the like, but is not limited thereto.

The present invention provides a transgenic organism transformed with the vector.

As used herein, "transformation" collectively refers to those processes in which genetic properties of a living organism are changed by injected DNA, "transgenic organism" refers to an organism prepared by injecting an external gene by a molecular genetic method and is preferably a cell or organism transformed by a recombinant expression vector of the present invention, but is not limited thereto.

In certain cases, the organism may include, without limitation, any living organism, such as a microorganism, a eukaryotic cell, an insect, an animal, or a plant, and is preferably *E. coli,* Salmonella, Bacillus, yeast, animal cells, a mouse, a rat, a dog, a monkey, a pig, a horse, a cow, *Agrobacterium tumefaciens,* a plant, and the like, but is not limited thereto.

The transgenic organism may be prepared by a method such as transformation, transfection, *Agrobacterium-*mediated transformation, particle gun bombardment, sonication, electroporation, and polyethylene glycol (PEG)-mediated transformation, but the method is not limited thereto. Furthermore, by a method of introducing the transgenic organism into cells, the intracellular uptake may be increased by introducing the transgenic organism with a delivery agent including G-fectin, a Mirus TransIT-TKO lipophilic agent, lipofectin, lipofectamine, cellfectin, cationic phospholipid nanoparticles, a cationic polymer, a cationic micelle, a cationic emulsion, or a liposome into cells or conjugating a biocompatible polymer such as polyethylene glycol, but the method is not limited thereto.

When the vector of the present invention is transformed into a eukaryotic cell, as host cells, yeast (*Saccharomyce cerevisiae*), insect cells, human cells (for example, a CHO cell line (Chinese hamster ovary), W138, BHK, COS-7, 293, HepG2, 3T3, RIN and MDCK cell lines), plant cells, and the like may be used, and preferably, *Agrobacterium* may be used.

When the host cell is a prokaryotic cell, the method of introducing the vector of the present invention into the host cell may be performed by a CaCl₂ method, the Hanahan method (Hanahan, D., J. Mol. Biol., 166:557-580 (1983)), an electroporation method, or the like. Further, when the host cell is a eukaryotic cell, the vector may be injected into the host cell by a microinjection method, a calcium phosphate precipitation method, an electroporation method, a liposome-mediated transfection method, a DEAE-dextran treatment method, gene bombardment, and the like.

The present invention provides a pharmaceutical composition for preventing or treating graft-versus-host disease, autoimmune diseases, or type 2 diabetes, including, as an active ingredient, an anti-DR3 antibody or an antigen-binding fragment thereof, which specifically binds to the death receptor 3 (DR3).

In an exemplary embodiment of the present invention, the antibody or the antigen-binding fragment may include a heavy chain set forth in an amino acid sequence of SEQ ID NO: 83 and a light chain set forth in an amino acid sequence of SEQ ID NO: 84, but is not limited thereto.

In an exemplary embodiment of the present invention, the autoimmune disease may be any one or more selected from the group consisting of an autoimmune disease caused by graft-versus-host disease, hemophagocytic lymphohistiocytosis, systemic lupus erythematosus, Kikuchi disease, adult onset Stills disease, Behcet disease, IgG4-related disease, type 1 diabetes, systemic sclerosis, psoriasis, multiple sclerosis, and Graves hyperthyroidism, but is not limited thereto.

The pharmaceutical composition of the present invention may further include an appropriate carrier, excipient, and diluent, which are typically used to prepare a pharmaceutical composition. The excipient may be, for example, one or more selected from the group consisting of a diluent, a binder, a disintegrant, a lubricant, an adsorbent, a moisturizer, a film-coating material, and a controlled release additive.

The pharmaceutical composition according to the present invention may be used by being formulated into the form of an external preparation such as a powder, a granule, a sustained-release granule, an enteric granule, a liquid, a collyrium, an elixir, an emulsion, a suspension, a spirit, a troche, aromatic water, a limonade, a tablet, a sustained-release tablet, an enteric tablet, a sublingual tablet, a hard capsule, a soft capsule, a sustained-release capsule, an enteric capsule, a pill, a tincture, a soft extract agent, a dry extract agent, a fluid extract agent, an injection, a capsule, a perfusate, a plaster, a lotion, a paste, a spray, an inhalant, a patch, a sterilized injection solution, or an aerosol, and the external preparation may have a formulation such as a cream, a gel, a patch, a spray, an ointment, a plaster, a lotion, a liniment, a paste or a cataplasma.

Examples of a carrier, an excipient or a diluent which may be included in the composition according to the present invention include lactose, dextrose, sucrose, an oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When the pharmaceutical composition is prepared, the pharmaceutical composition is prepared using a diluent or excipient, such as a filler, an extender, a binder, a wetting agent, a disintegrant, and a surfactant, which are commonly used.

As an additive of the tablet, powder, granule, capsule, pill, and troche according to the present invention, it is possible to use an excipient such as corn starch, potato starch, wheat starch, lactose, sucrose, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, carboxymethyl cellulose sodium, kaolin, urea, colloidal silica gel, hydroxypropyl starch, hydroxypropyl methylcellulose (HPMC) 1928, HPMC 2208, HPMC 2906, HPMC 2910, propylene glycol, casein, calcium lactate, or Primojel; and a binder such as gelatin, arabic gum, ethanol, agar powder, cellulose acetate phthalate, carboxymethyl cellulose, carboxymethyl cellulose calcium, glucose, purified water, sodium caseinate, glycerin, stearic acid, carboxymethyl cellulose sodium, methylcellulose sodium, methylcellulose, microcrystalline cellulose, dextrin, hydroxycellulose, hydroxypropyl starch, hydroxymethyl cellulose, purified shellac, starch, hydroxypropyl cellulose, hydroxypropyl methyl cellulose, polyvinyl alcohol, or polyvinylpyrrolidone, and it is possible to use a disintegrant such as hydroxypropyl methyl cellulose, corn starch, agar powder, methyl cellulose, bentonite, hydroxypropyl starch, carboxymethyl cellulose sodium, sodium alginate, carboxymethyl cellulose calcium, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, arabic gum, amylopectin, pectin, sodium polyphosphate, ethyl cellulose, sucrose, magnesium aluminum silicate, a D-sorbitol solution, or light anhydrous silicic acid; and a lubricant such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol 4000, PEG 6000, liquid paraffin, hydrogenated soybean oil (Lubriwax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, or light anhydrous silicic acid.

As an additive for liquid formulation according to the present invention, it is possible to use water, diluted hydrochloric acid, diluted sulfuric acid, sodium citrate, sucrose monostearate, polyoxyethylene sorbitol fatty acid esters (Tween esters), polyoxyethylene monoalkyl ethers, lanolin ether, lanolin esters, acetic acid, hydrochloric acid, aqueous ammonia, ammonium carbonate, potassium hydroxide, sodium hydroxide, prolamin, polyvinyl pyrrolidone, ethyl cellulose, carboxymethyl cellulose sodium, and the like.

In a syrup according to the present invention, a solution of sucrose, other sugars or sweeteners, and the like may be used, and a fragrance, a colorant, a preservative, a stabilizer, a suspending agent, an emulsifier, a thickener, and the like may be used if necessary.

Purified water may be used for the emulsion according to the present invention, and an emulsifier, a preservative, a stabilizer, a fragrance, and the like may be used if necessary.

In the suspension according to the present invention, a suspending agent such as acacia, tragacanth, methyl cellulose, carboxymethyl cellulose, carboxymethyl cellulose sodium, microcrystalline cellulose, sodium alginate, hydroxypropyl methyl cellulose, HPMC 1828, HPMC 2906, and HPMC 2910 may be used, and a surfactant, a preservative, a colorant, and a fragrance may be used if necessary.

The injection according to the present invention may include: a solvent such as distilled water for injection, a 0.9% sodium chloride injection, a Ringer's injection, a dextrose injection, a dextrose+sodium chloride injection, PEG, a lactated Ringer's injection, ethanol, propylene glycol, non-volatile oil-sesame oil, cottonseed oil, peanut oil, corn oil, ethyl oleate, isopropyl myristate, or benzoic acid benzene; a solubilizing aid such as sodium benzoate, sodium salicylate, sodium acetate, urea, urethane, monoethyl acetamide, butazolidin, propylene glycol, Tweens, nijungtinateamide, hexamine, or dimethylacetamide; a buffer such as a weak acid or salt thereof (acetic acid or sodium acetate), a weak base or salt thereof (ammonia or ammonium acetate), an organic compound, a protein, albumin, peptone, or a gum; an isotonic agent such as sodium chloride; a stabilizer such as sodium bisulfite (NaHSO₃), carbon dioxide gas, sodium metabisulfite (Na₂S₂O₅), sodium sulfite (Na₂SO₃), nitrogen gas (N₂), or ethylenediaminetetraacetic acid; a sulfating agent such as 0.1% sodium bisulfide, sodium formaldehydesulfoxylate, thiourea, disodium ethylenediaminetetraacetate, or acetone sodium bisulfite; an analgesic such as benzyl alcohol, chlorobutanol, procaine hydrochloride, glucose, or calcium gluconate; and a suspending agent such as carboxymethyl cellulose sodium, sodium alginate, Tween 80, or aluminum monostearate.

In a suppository according to the present invention, it is possible to use a base such as cacao butter, lanolin, Witepsol, polyethylene glycol, glycerogelatin, methylcellulose, carboxymethyl cellulose, a mixture of stearic acid and oleic acid, Subanal, cottonseed oil, peanut oil, palm oil, cacao butter+cholesterol, lecithin, ranetwax, glycerol monostearate, Tween or Span, Imhausen, monolen (propylene glycol monostearate), glycerin, Adeps solidus, Buytyrum Tego-G, Cebes Pharma 16, hexalide base 95, Cotomar, Hydrokote SP, S-70-XXA, S-70-XX75(S-70-XX95), Hydrokote 25, Hydrokote 711, idropostal, Massa estrarium (A, AS, B, C, D, E, I, T), Massa-MF, Masupol, Masupol-15, Neosupostal-ene, Paramound-B, Suposhiro (OSI, OSIX, A, B, C, D, H, L), suppository base IV types (AB, B, A, BC, BBG, E, BGF, C, D, 299), Supostal (N, Es), Wecobee (W, R, S, M ,Fs), and Tegester triglyceride bases (TG-95, MA, 57).

A solid formulation for oral administration includes a tablet, a pill, a powder, a granule, a capsule, or the like, and the solid formulation is prepared by mixing at least one excipient, for example, starch, calcium carbonate, sucrose or lactose, gelatin, and the like, with an extract. Further, in addition to a simple excipient, lubricants such as magnesium stearate and talc are also used.

A liquid formulation for oral administration corresponds to a suspension, a liquid for internal use, an emulsion, a syrup, or the like, and the liquid formulation may include, in addition to water and liquid paraffin, which are simple commonly used diluents, various excipients, for example, a wetting agent, a sweetener, a fragrance, a preservative, and the like. Examples of a formulation for parenteral administration include an aqueous sterile solution, a non-aqueous solvent, a suspension, an emulsion, a freeze-dried preparation, and a suppository. As the non-aqueous solvent and the suspending agent, it is possible to use propylene glycol, polyethylene glycol, a vegetable oil such as olive oil, an injectable ester such as ethyl oleate, and the like.

The pharmaceutical composition according to the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including the type of disease of a patient, the severity of the disease, the activity of drugs, sensitivity to drugs, administration time, administration route, excretion rate, treatment period, and simultaneously used drugs, and other factors well known in the medical field.

The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by a person with ordinary skill in the art to which the present invention pertains.

The pharmaceutical composition of the present invention may be administered to a subject in need via various routes. All methods of administration may be expected, but the pharmaceutical composition may be administered by, for example, oral administration, subcutaneous injection, peritoneal administration, intravenous injection, intramuscular injection, paraspinal space (intradural) injection, sublingual administration, buccal administration, intrarectal insertion, intravaginal injection, ocular administration, ear administration, nasal administration, inhalation, spraying via the mouth or nose, skin administration, transdermal administration, and the like.

The pharmaceutical composition of the present invention is determined by the type of drug that is an active ingredient, as well as various related factors such as the disease to be treated, the route of administration, the age, sex, and body weight of a patient, and the severity of the disease. Specifically, the effective amount of the composition according to the present invention may vary depending on the patient's age, sex, and body weight, and generally, 0.001 to 150 mg of the composition, and preferably 0.01 to 100 mg of the composition, per 1 kg of body weight may be administered daily or every other day or administered once to three times a day. However, since the preferred dosage may be increased or decreased depending on the administration route, the severity of disease, the sex, the body weight, the age, and the like, the preferred dosage is not intended to limit the scope of the present invention by any method.

As used herein, "subject" refers to a subject in need of treatment of a disease, and more specifically, refers to a mammal such as a human or a non-human primate, a mouse, a rat, a dog, a cat, a horse, and a cow.

As used herein, "administration" refers to the provision of a predetermined composition of the present invention to a subject in need thereof by any suitable method. As used herein, "prevention" refers to all actions that suppress or delay the onset of a target disease, and "treatment" refers to all actions that ameliorate or beneficially change a target disease and the resulting metabolic abnormalities by administration of the pharmaceutical composition according to the present invention, and "amelioration" refers to all actions that reduce a target disease and associated parameters, for example, the severity of symptoms, by administration of the composition according to the present invention.

The present invention provides a kit for preventing or treating graft-versus-host disease, autoimmune diseases, or type 2 diabetes, including the vector or the composition, and instructions.

In addition to the above configuration, the kit of the present invention may include other components, devices, substances, and the like that are typically required for a method for expressing the vector of the present invention, and for storing, managing, and enhancing the effect of the composition of the present invention. In addition, all configurations included in the kit may be used one or more times without any limitations, there is no limitation on the order in which each substance is used, and the application of each substance may be carried out simultaneously or in small increments.

The kit of the present invention may include a container in addition to the formulation and instructions. The container may serve to package the configuration, and may also serve to store and fix the same. A material for the container may take the form of, for example, a bottle, tub, sachet, envelope, tube, ampoule, and the like, which may be partially or entirely made of plastic, glass, paper, foil, wax, and the like. The container may be fitted with a fully or partially detachable stopper that may be initially part of the container or that may be attached to the container by mechanical, adhesive, or other means, and may be fitted with a stopper that allows access to the contents by an injection needle. The kit may include an external package, and the external package may include instructions for use of the constitutional elements, but is not limited thereto.

Hereinafter, preferred examples for helping with understanding of the present invention will be suggested. However, the following examples are provided only so that the present invention may be more easily understood, and the content of the present invention is not limited by the following examples.

### [Examples]

### Example 1. Discovery of anti-DR3 clone using phage display

A clone that specifically binds to DR3 was discovered using a phage display. First, scFv clones that bind to DR3 were selectively amplified through three rounds of panning using a human scFv library and recombinant DR3. It was confirmed that at each stage, scFv clones that bind to recombinant DR3 were amplified by ELISA using a phage obtained after panning. Thereafter, a single clone confirmed by colony PCR was infected with the phage, a single clone binding to DR3 was confirmed by ELISA, and the CDR region of each single clone was confirmed by sequencing.

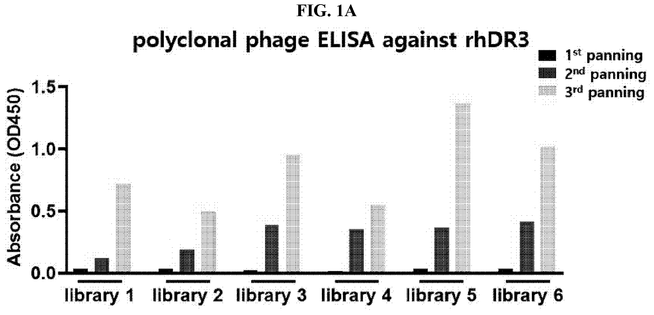

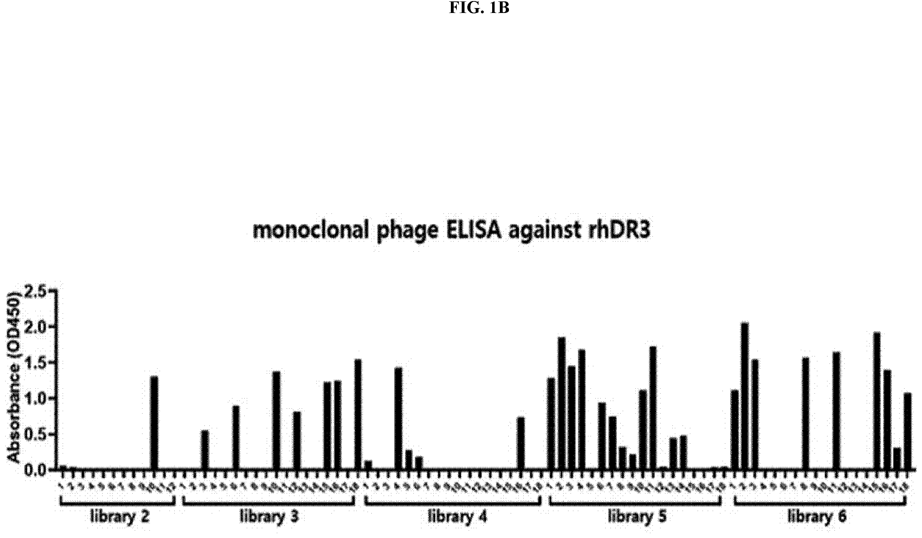

As a result, five clones binding to DR3 were discovered (FIGS. 1A and 1B), and the five types of antibodies were named 2-10, 3-18, 4-4, 6-2, and 6-15. Five types of antibodies were sequenced, and the complementarity determining region (CDR) of each clone was sequenced. Tables 1 and 2 show the amino acid and base sequences of CDR1 to CDR3 of the heavy chain region and variable region of each antibody. Table 3 shows the base sequence and amino acid sequence of the heavy chain region and variable region of each antibody. In each table, the bold text indicates the CDR sequence according to IMGT, and the underlined part indicates the CDR sequence according to KABAT.

**[Table 1]**

| **SEQ ID NO:** | **Name** | **Sequence** |
|---|---|---|
| SEQ ID NO:1 | Heavy chain CDR1 of 2-10 mAb variable region | **GYTF****SYG**IS |
| SEQ ID NO:2 | Heavy chain CDR2 of 2-10 mAb variable region | W**ISAYHGNT**NYAQKLQG |
| SEQ ID NO:3 | Heavy chain CDR3 of 2-10 mAb variable region | **ASGPDYGMLDY** |
| SEQ ID NO:4 | Light chain CDR1 of 2-10 mAb variable region | QAS**QDISNY** |
| SEQ ID NO:5 | Light chain CDR2 of 2-10 mAb variable region | **DAS**NLET |
| SEQ ID NO:6 | Light chain CDR3 of 2-10 mAb variable region | **QQYINLPLT** |
| SEQ ID NO:7 | Heavy chain CDR1 of 3-18 mAb variable region | **GGSIS****SSSYY**WG |
| SEQ ID NO:8 | Heavy chain CDR2 of 3-18 mAb variable region | S**IYYSGST**YYNPSLKS |
| SEQ ID NO:9 | Heavy chain CDR3 of 3-18 mAb variable region | **ABQVSGPLDY** |
| SEQ ID NO:10 | Light chain CDR1 of 3-18 mAb variable region | RAS**OGIBND**LG |
| SEQ ID NO:11 | Light chain CDR2 of 3-18 mAb variable region | **AAS**SLQS |
| SEQ ID NO:12 | Light chain CDR3 of 3-18 mAb variable region | **LQDYNYPRT** |
| SEQ ID NO:13 | Heavy chain CDR1 of 4-4 mAb variable region | **GFTFS****DYYM**S |
| SEQ ID NO:14 | Heavy chain CDR2 of 4-4 mAb variable region | Y**ISSSSSYT**NYADSVKG |
| SEQ ID NO:15 | Heavy chain CDR3 of 4-4 mAb variable region | **ASNVDTAMVGAFDI** |
| SEQ ID NO:16 | Light chain CDR1 of 4-4 mAb variable region | RAS**QHISNV**LA |
| SEQ ID NO:17 | Light chain CDR2 of 4-4 mAb variable region | **TAS**HLQS |
| SEQ ID NO:18 | Light chain CDR3 of 4-4 mAb variable region | **QQADSFPLT** |
| SEQ ID NO:19 | Heavy chain CDR1 of 6-2 mAb variable region | **GFTFS****SYS**MN |
| SEQ ID NO:20 | Heavy chain CDR2 of 6-2 mAb variable region | Y**ISSSSST**IYYADSVIKG |
| SEQ ID NO:21 | Heavy chain CDR3 of 6-2 mAb variable region | **ARWDGYCSGGSCYSGMDY** |
| SEQ ID NO:22 | Light chain CDR1 of 6-2 mAb variable region | QAS**QDISNY**LN |
| SEQ ID NO:23 | Light chain CDR2 of 6-2 mAb variable region | **DA**SNLET |
| SEQ ID NO:24 | Light chain CDR3 of 6-2 mAb variable region | **QQYINLPLT** |
| SEQ ID NO:25 | Heavy chain CDR1 of 6-15 mAb variable region | **GFTFS****SYS**MN |
| SEQ ID NO:26 | Heavy chain CDR2 of 6-15 mAb variable region | Y**ISSSSST**IYYADSVKG |
| SEQ ID NO:27 | Heavy chain CDR3 of 6-15 mAb variable region | **ABWDGYCSGGSCYSGMDY** |
| SEQ ID NO:28 | Light chain CDR1 of 6-15 mAb variable region | QAS**QDISNY**LN |
| SEQ ID NO: 29 | Light chain CDR2 of 6-15 mAb variable region | **DAS**NLET |
| SEQ ID NO: 30 | Light chain CDR3 of 6-15 mAb variable region | **QQYINLPLT** |

**[Table 2]**

| **SEQ ID NO:** | **Name** | **Sequence** |
|---|---|---|
| SEQ ID NO:31 | Heavy chain CDR1 of 2-10 mAb variable region | GGTTACACCTTTACCAGCTATGGTATCAGC |
| SEQ ID NO:32 | Heavy chain CDR2 of 2-10 mAb variable region | TGGATCAGCGCTTACAATGGTAACACAAACTATGCACAGAAGC TCCAGGGC |
| SEQ ID NO:33 | Heavy chain CDR3 of 2-10 mAb variable region | GCGAGTGGCCCCGACTACGGCATGCTTGACTAC |
| SEQ ID NO:34 | Light chain CDR1 of 2-10 mAb variable region | CAGGCGAGTCAGGACATTAGCAACTAC |
| SEQ ID NO:35 | Light chain CDR2 of 2-10 mAb variable region | GATGCATCCAATTTGGAAACA |
| SEQ ID NO:36 | Light chain CDR3 of 2-10 mAb variable region | CAACAGTATGATAATCTCCCCCTCACT |
| SEQ ID NO:37 | Heavy chain CDR1 of 3-18 mAb variable region | GGTGGCTCCATCAGCAGTAGTAGTTACTACTGGGGC |
| SEQ ID NO:38 | Heavy chain CDR2 of 3-18 mAb variable region | AGTATCTATTATAGTGGGAGCACCTACTACAACCCGTCCCTCA AGAGT |
| SEQ ID NO:39 | Heavy chain CDR3 of 3-18 mAb variable region | GCGAGGCAAGTTTCGGGGCCCCTTGACTAC |
| SEQ ID NO:40 | Light chain CDR1 of 3-18 mAb variable region | CGGGCAAGTCAGGGCATTAGAAATGATTTAGGC |
| SEQ ID NO:41 | Light chain CDR2 of 3-18 mAb variable region | GCT GCATCCAGTTTACAAAGT |
| SEQ ID NO:42 | Light chain CDR3 of 3-18 mAb variable region | CTACAAGATTACAATTACCCTCGGACG |
| SEQ ID NO:43 | Heavy chain CDR1 of 4-4 mAb variable region | GGATTCACCTTCAGTGACTACTACATGAGT |
| SEQ ID NO:44 | Heavy chain CDR2 of 4-4 mAb variable region | TACATTAGTAGTAGTAGTAGTTACACAAACTACGCAGACTCT[?] TGAAGGGC |
| SEQ ID NO:45 | Heavy chain CDR3 of 4-4 mAb variable region | GCGAGTAACGTGSATACAGCTATGGTTGGGGCTTTTGATATC |
| SEQ ID NO:46 | Light chain CDR1 of 4-4 mAb variable region | CGGGCGAGTCAACATATTAGCAACTGGTTAGCC |
| SEQ ID NO:47 | Light chain CDR2 of 4-4 mAb variable region | ACTGCATCCAATTTGCAAAGT |
| SEQ ID NO:48 | Light chain CDR3 of 4-4 mAb variable region | CAACAGGCTGACAGTTTCCCGCTCACT |
| SEQ ID NO:49 | Heavy chain CDR1 of 6-2 mAb variable region | GGATTCACCTTCAGTAGCTATAGCATGAAC |
| SEQ ID NO:50 | Heavy chain CDR2 of 6-2 mAb variable region | TACATTAGTAGTAGTAGTAGCACCATATACTACGCAGACTCTG TGAAGGGC |
| SEQ ID NO:51 | Heavy chain CDR3 of 6-2 mAb variable region | TACATTAGTAGTAGTAGTAGCACCATATACTACGCAGACTCTG TGAAGGGC |
| SEQ ID NO:52 | Light chain CDR1 of 6-2 mAb variable region | CAGGCGAGTCAGGACATTAGCAACTATTTAAAT |
| SEQ ID NO:53 | Light chain CDR2 of 6-2 mAb variable region | GATGCATCCAATTTGGAAACA |
| SEQ ID NO:54 | Light chain CDR3 of 6-2 mAb variable region | CAACAGTATGATAATCTCCCCCTCACT |
| SEQ ID NO:55 | Heavy chain CDR1 of 6-15 mAb variable region | GGATTCACCTTCAGTAGCTATAGCATGAAC |
| SEQ ID NO:56 | Heavy chain CDR2 of 6-15 mAb variable region | TACATTAGTAGTAGTAGTAGCACCATATACTACGCAGACTCTG TGAAGGGC |
| SEQ ID NO:57 | Heavy chain CDR3 of 6-15 mAb variable region | GCGAGATGGGATGGATATTGTAGTGGTGGTAGCTGCTACTCCG GGATGGACTAC |
| SEQ ID NO:58 | Light chain CDR1 of 6-15 mAb variable region | CAGGCGAGTCAGGACATTAGCAACTATTTAAAT |
| SEQ ID NO:59 | Light chain CDR2 of 6-15 mAb variable region | GATGCATCCAATTTGGAAACA |
| SEQ ID NO:60 | Light chain CDR3 of 6-15 mAb variable region | CAACAGTATGATAATCTCCCTCTCACT |

**[Table 3]**

| **SEQ ID NO:** | **Name** | **Sequence** |
|---|---|---|
| SEQ ID NO:61 | DNA sequence heavy chain of 2-10 mAb variable region | CAGGTGCAGCTGGTGCAGTCTGGAGCTGAGGTGAAGAAGCCTG GGGCCTCAGTGAAGGTCTCCTGCAAGGCTTCTGGTTACACCTT TACCAGCTATGGTATCAGCTGGGTGCGACAGGCCCCTGGACAA GGGCTTGAGTGGATGGGATGGATCAGCGCTTACAATGGTAACA CAAACTATGCACAGAAGCTCCAGGGCAGAGTCACCATGACCAC AGACACATCCACGAGCACAGCCTACATGGAGCTGAGGAGCCTG AGATCTGACGACACGGCCGTGTATTACTGTGCGAGTGGCCCCG ACTACGGCATGCTTGACTACTGGGGCCAGGGCACCCTGGTCAC CGTCTCC |
| SEQ ID NO:62 | DNA sequence light chain of 2-10 mAb variable region | GAAATTGTGATGACGCAGTCTCCATCCTCCCTGCCTGCATCTG TAGGAGACAGAGTCACCATCACTTGCCAGGCGAGTCAGGACAT TAGCAACTACTTAAATTGGTATCAGCAGAAACCAGGGAAAGCC CCTAAGCTCCTGATCTACGATGCATCCAATTTGGAAACAGGGG TCCCATCAAGGTTCAGTGGAAGTGGATCTGGGACAGATTTTAC TTTCACCATCAGCAGCCCGCAGCCTGAAGATATTGCAACATAT TACTGTCAACAGTATGATAATCTCCCCCTCACTTTCGGCGGAG GGACCAAGGTGGAGATCAAA |
| SEQ ID NO:63 | Amino acid sequence heavy chain of 2-10 mAb variable region | QVQLVQSGAEVKKPGASVKVSCKASGYTFTSYGISWVRQAPGQ GLEWMGWISAYNGNTNYAQKLQGRVTMTTDTSTSTAYMELRSL RSDDTAVYYCASGPDYGMLDYWGQGTLVTVS |
| SEQ ID NO:64 | Amino acid sequence light chain of 2-10 mAb variable region | EIVMTQSPSSLPASVGDRVTITCQASQDISNYLNWYQQKPGKA PKLLIYDASNLETGVPSRFSGSGSGTDFTFTISSPQPEDIATY YCQQYDNLPLTFGGGTKVEIK |
| SEQ ID NO:65 | DNA sequence heavy chain of 3-18 mAb variable region | CAGGTGCAGCTGCAGCAGTCGGGCCCAGGACTGGTGAAGCCTT CGGAGACCCTGTCCCTCACCTGCACTGTCTCTGGTGGCTCCAT CAGCAGTAGTAGTTACTACTGGGGCTGGATCCGCCAGCCCCCA GGGAAGGGGCTGGAGTGGATTGGGAGTATCTATTATAGTGGGA GCACCTACTACAACCCGTCCCTCAAGAGTCGAGTCACCATATC CGTAGACACGTCCAAGAACCAGTTCTCCCTGAAGCTGAGCTCT GTGACCGCCGCAGACACGGCTGTGTATTACTGTGCGAGGCAAG TTTCGGGGCCCCTTGACTACTGGGGCCAGGGGACCCTGGTCAC CGTCTCC |
| SEQ ID NO:66 | DNA sequence light chain of 3-18 mAb variable region | GACATCGTGATGACCCAGTCTCCATCCTCCCTGTCTGCATCTG TAGGAGACAGAGTCACCATCACTTGCCGGGCAAGTCAGGGCAT TAGAAATGATTTAGGCTGGTATCAGCAGAAACCAGGGAAAGCC CCTAAGCTCCTGATCTATGCTGCATCCAGTTTACAAAGTGGGG TCCCATCAAGGTTCAGCGGCAGTGGATCTGGCACAGATTTCAC TCTCACCATCAGCAGCCTGCAGCCTGAAGATTTTGCAACTTAT TACTGTCTACAAGATTACAATTACCCTCGGACGTTCGGCCAAG GGACCAAGGTGGAAATCAAA |
| SEQ ID NO:67 | Amino acid sequence heavy chain of 3-18 mAb variable region | QVQLQQSGPGLVKPSETLSLTCTVSGGSISSSSYYWGWIRQPP GKGLEWIGSIYYSGSTYYNPSLKSRVTISVDTSKNQFSLKLSS VTAADTAVYYCARQVSGPLDYWGQGTLVTVS |
| SEQ ID NO:68 | Amino acid sequence light chain of 3-18 mAb variable region | DIVMTQSPSSLSASVGDRVTITCRASQGIRNDLGWYQQKPGKA PKLLIYAASSLQSGVPSRFSGSGSGTDFTLTISSLQPEDFATY YCLQDYNYPRTFGQGTKVEIK |
| SEQ ID NO:69 | DNA sequence heavy chain of 4-4 mAb variable region | GAGGTGCAGCTGGTGGAGTCTGGGGGAGGCTTGGTCAAGCCTG GAGGGTCCCTGAGACTCTCCTGTGCAGCCTCTGGATTCACCTT CAGTGACTACTACATGAGCTGGATCCGCCAGGCTCCAGGGAAG GGGCTGGAGTGGGTTTCATACATTAGTAGTAGTAGTAGTTACA CAAACTACGCAGACTCTGTGAAGGGCCGATTCACCATCTCCAG AGACAACGCCAAGAACTCACTGTATCTGCAAATGAACAGCCTG AGAGCCGAGGACACGGCCGTGTATTACTGTGCGAGTAACGTGG ATACAGCTATGGTTGGGGCTTTTGATATCTGGGGCCAAGGAAC CCTGGTCACCGTCTCC |
| SEQ ID NO:70 | DNA sequence light chain of 4-4 mAb variable region | GACATCGAGATGACCCAGTCTCCTTCTTCCGTGTCTGCATCTG TAGGAGACACAGTCACCATCACCTGCCGGGCGAGTCAACATAT TAGCAACTGGTTAGCCTGGTATCAGCAGAAACCAGGGCAAGCC CCTAAGTTCCTGATCTATACTGCATCCAATTTGCAAAGTGGGG TCCCATCAAGATTCAGCGGCAGTGGATATGGGACAGATTTCAC TCTCACCATCAGCGGCCTGCAGCCTGAAGATTTTGCGACTTAC TATTGTCAACAGGCTGACAGTTTCCCGCTCACTTTCGGCGGAG GGACCAAGGTGGATATCAAA |
| SEQ ID NO:71 | Amino acid sequence heavy chain of 4-4 mAb variable region | EVQLVESGGGLVKPGGSLRLSCAASGFTFSDYYMSWIRQAPGK GLEWVSYISSSSSYTNYADSVKGRFTISRDNAKNSLYLQMNSL RAEDTAVYYCASNVDTAMVGAFDIWGQGTLVTVS |
| SEQ ID NO:72 | Amino acid sequence light chain of 4-4 mAb variable region | DI EMTQSPSSVSASVGDTVTITCRASQHI SNWLAWYQQKPGQA PKFLIYTASNLQSGVPSRFSGSGYGTDFTLTISGLQPEDFATY YCQQADSFPLTFGGGTKVDI K |
| SEQ ID NO:73 | DNA sequence heavy chain of 6-2 mAb variable region | CAGGTGCAGCTGGTGCAGTCTGGGGGAGGCTTGGTACAGCCTG GGGGGTCCCTGAGACTCTCCTGTGCAGCCTCTGGATTCACCTT CAGTAGCTATAGCATGAACTGGGTCCGCCAGGCTCCAGGGAAG GGGCTGGAGTGGGTTTCATACATTAGTAGTAGTAGTAGCACCA TATACTACGCAGACTCTGTGAAGGGCCGATTCACCATCTCCAG AGACAACGCCAAGAACTCACTGTATCTGCAAATGAACAGCCTG AGAGCCGAGGACACGGCTGTGTATTACTGTGCGAGATGGGATG GATATTGTAGTGGTGGTAGCTGCTACTCCGGGATGGACTACTG GGGCCAGGGCACCCTGGTCACCGTCTCC |
| SEQ ID NO:74 | DNA sequence light chain of 6-2 mAb variable region | GACATCGAGATGACCCAGTCTCCATCCTCCCTGTCTGCATCTG TAGGAGACAGAGTCACCATCACTTGCCAGGCGAGTCAGGACAT TAGCAACTATTTAAATTGGTATCAGCAGAAACCAGGGAAAGCC CCTAAGCTCCTGATCTACGATGCATCCAATTTGGAAACAGGGG TCCCATCAAGGTTCAGTGGAAGTGGATCTGGGACAGATTTTAC TTTCACCATCAGCAGCCTGCAGCCTGAAGATATTGCAACATAT TACTGTCAACAGTATGATAATCTCCCCCTCACTTTCGGCGGAG GGACCAAGGTGGAGATCAAA |
| SEQ ID NO:75 | Amino acid sequence heavy chain of 6-2 mAb variable region | QVQLVQSGGGLVQPGGSLRLSCAASGFTFSSYSMNWVRQAPGK GLEWVSYISSSSSTIYYADSVKGRFTISRDNAKNSLYLQMNSL RAEDTAVYYCARWDGYCSGGSCYSGMDYWGQGTLVTVS |
| SEQ ID NO:76 | Amino acid sequence light chain of 6-2 mAb variable region | DIEMTQSPSSLSASVGDRVTITCQASQDISNYLNWYQQKPGKA PKLLIYDASNLETGVPSRFSGSGSGTDFTFTISSLQPEDIATY YCQQYDNLPLTFGGGTKVEIK |
| SEQ ID NO:77 | DNA sequence heavy chain of 6-15 mAb variable region | CAGGTGCAGCTGGTGCAGTCTGGGGGAGGCTTGGTACAGCCTG GGGGGTCCCTGAGACTCTCCTGTGCAGCCTCTGGATTCACCTT CAGTAGCTATAGCATGAACTGGGTCCGCCAGGCTCCAGGGAAG GGGCTGGAGTGGGTTTCATACATTAGTAGTAGTAGTAGCACCA TATACTACGCAGACTCTGTGAAGGGCCGATTCACCATCTCCAG AGACAACGCCAAGAACTCACTGTATCTGCAAATGAACAGCCTG AGAGCCGAGGACACGGCTGTGTATTACTGTGCGAGATGGGATG GATATTGTAGTGGTGGTAGCTGCTACTCCGGGATGGACTACTG GGGCCAGGGCACCCTGGTCACCGTCTCC |
| SEQ ID NO:78 | DNA sequence light chain of 6-15 mAb variable region | GACATCGAGATGACCCAGTCTCCATCCTCCCTGTCTGCATCTG TAGGAGACAAAGTCACCATCACTTGCCAGGCGAGTCAGGACAT TAGCAACTATTTAAATTGGTATCAGCAGAAACCAGGGAAAGCC CCTAAGCTCCTGATCTACGATGCATCCAATTTGGAAACAGGGG TCCCATCAAGGTTCAGTGGAAGTGGATCTGGGACAGATTCTAC TTTCACCATCAGCAGCCTGCAGCCTGAAGATATTGCAACATAT TACTGTCAACAGTATGATAATCTCCCTCTCACTTTCGGCGGAG GGACCAAGGTGGAGATCAAA |
| SEQ ID NO:79 | Amino acid sequence heavy chain of 6-15 mAb variable region | QVQLVQSGGGLVQPGGSLRLSCAASGFTFSSYSMNWVRQAPGK GLEWVSYISSSSSTIYYADSVKGRFTISRDNAKNSLYLQMNSL RAEDTAVYYCARWDGYCSGGSCYSGMDYWGQGTLVTVS |
| SEQ ID NO:80 | Amino acid sequence light chain of 6-15 mAb variable region | DIEMTQSPSSLSASVGDKVTITCQASQDISNYLNWYQQKPGKA PKLLIYDASNLETGVPSRFSGSGSGTDSTFTISSLQPEDIATY YCQQYDNLPLTFGGGTKVEIK |

### Example 2. Confirmation of excellent DR3-specific binding strength of five types of antibodies

The specific binding strength of the five types of antibodies developed in Example 1 to DR3 was confirmed through ELISA, SPR, and FACS experiments.

### Example 2-1. Confirmation of specific binding strength to DR3 using ELISA

ELISA was performed using monoclonal antibodies from the clones selected in Example 1. Specifically, 0.4 µg/ml DR3-His tag was put into and mixed with PBS, and then a Ni plate was coated with a total of 50 µl of the mixed solution. Thereafter, to prevent non-specific binding, the Ni plate was treated with 200 µl of PBS containing 5% FBS and incubated at room temperature for 30 minutes, and incubated for 30 minutes by titrating the concentration of the monoclonal antibody. The degree of binding between the coated DR3 and the monoclonal antibody was detected with anti-human IgG Fc HRP. For each step, the plate was washed five times with 250 µl of 0.1% Tween 20. Isotype control was used as a negative control.

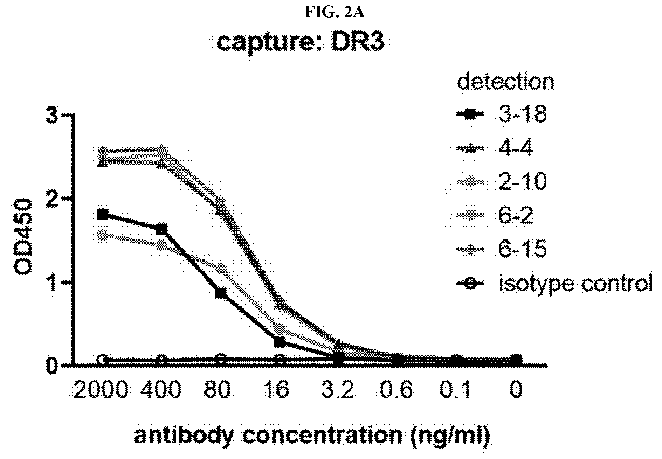

As a result, it was confirmed that the five antibodies of the present invention have excellent specific binding ability to DR3 compared to the control, and among them, 4-4, 6-2, and 6-15 maintain remarkably high binding specificity to DR3 even at low antibody concentrations (FIG 2A).

### Example 2-2. Confirmation of specific binding strength to DR3 using SPR

SPR was performed using monoclonal antibodies from the clones selected in Example 1. After immobilizing 10 µg/ml DR3-His tag on a CM5 chip, the binding specificity and binding strength were measured using Biacore T100 under the conditions of a contact time of 30 sec and a flow rate of 30 µl/min.

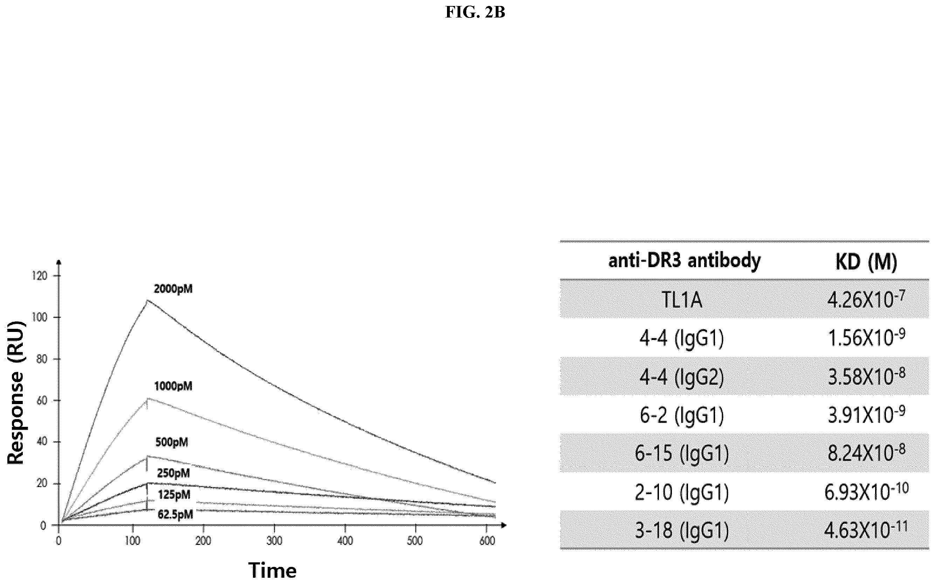

As a result, the five types of antibodies according to Example 1 were found to have remarkably lower binding dissociation constants for DR3 compared to TL1A, a DR3 agonist, and were confirmed to exhibit a binding strength of about 12-fold to 9,200-fold or more (FIG 2B).

### Example 2-3. Confirmation of specific binding strength to DR3 using FACS

FACS was performed using monoclonal antibodies from the clones selected in Example 1. Specifically, a DR3 stable cell line at a concentration of 0.2×10⁶ was mixed with 2 µg/ml monoclonal antibody and incubated at 4 °C for 30 minutes, and the degree of binding between the DR3 stable cell line and the monoclonal antibody was confirmed by FACS analysis using anti-human IgG PE. For each step, cells were washed once with 200 µl of PBS. Isotype control was used as a negative control.

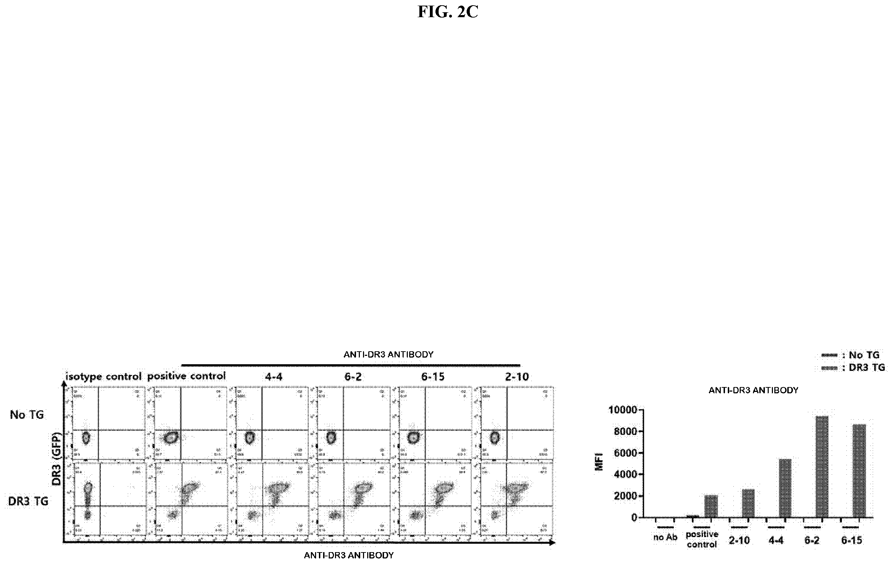

As a result, it was confirmed that antibodies 4-4, 6-2, and 6-15 strongly bind to DR3 (FIG 2C).

### Example 3. Epitope mapping of five antibodies against DR3

### Example 3-1. Epitope mapping using ELISA

ELISA was performed to confirm whether the epitope of rDR3 to which the anti-DR3 4-4 antibody binds among the five types of antibodies in Example 1 overlaps with TL1A, a ligand of rDR3, or other monoclonal antibodies. Specifically, 0.4 µg/ml DR3-His tag was put into and mixed with PBS, and then a Ni plate was coated with a total of 50 µl of the mixed solution. Thereafter, to prevent non-specific binding, the Ni plate was mixed with 200 µl of PBS consisting of 5% FBS and incubated at room temperature for 30 minutes, and incubated for 30 minutes by titrating the concentration of TL1A and the monoclonal antibody. Thereafter, 200 ng/ml of the biotinylated anti-DR3 4-4 antibody was incubated for 30 minutes without washing, and the degree of binding between DR3 and the biotinylated anti-DR3 4-4 antibody was detected with anti-human IgG Fc HRP. Except for the step without washing, the plate was washed five times with 250 µl of 0.1% Tween 20 at each step. Isotype control was used as a negative control.

### Example 3-2. Epitope mapping using FACS

FACS was performed to confirm whether the epitope in which the five selected antibodies bind to DR3 expressed on the cell surface overlaps with TL1A, a ligand of human DR3, or other monoclonal antibodies. A DR3 stable cell line at a concentration of 0.2×10⁶ was mixed with 2 µg/ml monoclonal antibody and incubated at 4 °C for 30 minutes, and mixed with 0.625 µg/ml TL1A His tag without washing, and the resulting mixture was incubated at 4 °C for 30 minutes. The degree of binding between the DR3 stable cell line and TL1A-His tag was detected using anti-His tag AF647 and confirmed by FACS. Except for the step without washing, cells were washed once with 200 µl of PBS at each step. Isotype control was used as a negative control.

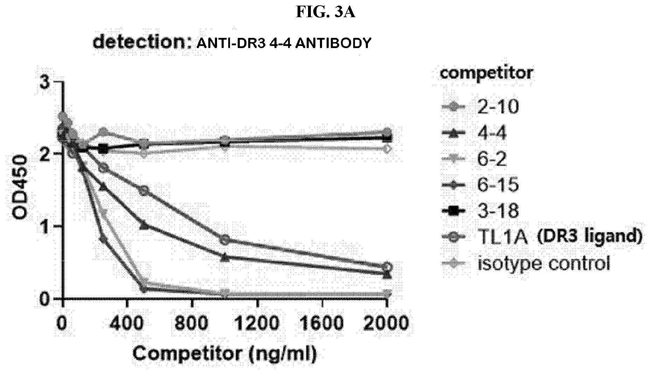

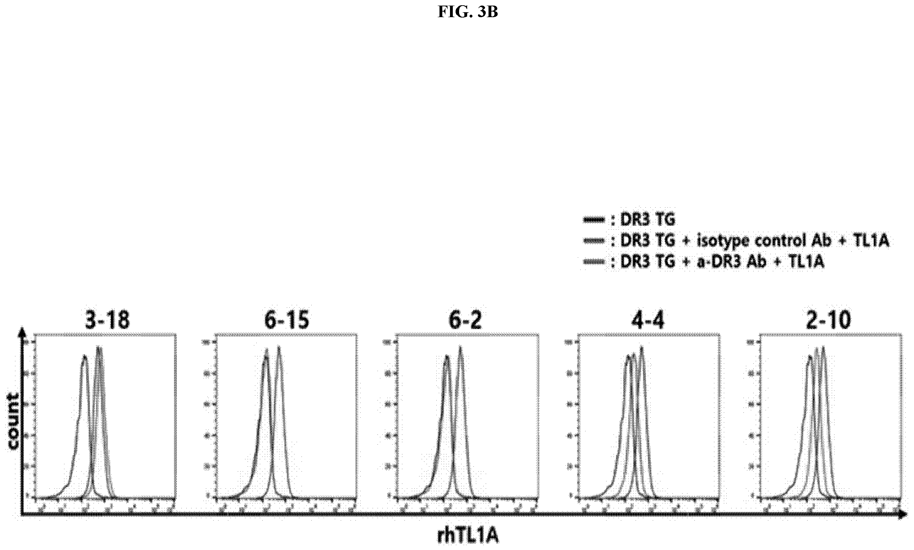

As a result, it was confirmed that clones 4-4, 6-2, and 6-15 bind to the epitope to which DR3 and TL1A bind, whereas clones 2-10 and 3-18 do not bind to the epitope (FIGS. 3A and 3B).

### Example 4. Confirmation of excellent NF-κB activity effect by selected antibodies

To confirm the NF-κB activity effect of the antibodies selected in Example 1, the phosphorylated NF-κB (pNF-κB) levels were compared and analyzed by FACS. Specifically, pNF-κB was activated with clone 4-4 IgG1, clone 2-10 IgG1, clone 3-18 IgG1, and clone 6-2 IgG1 monoclonal antibodies and rhTL1A. 1×10⁶ hPBMCs were mixed with rhTL1A-His tag or the titrated monoclonal antibody, and then incubated at 37 °C for 10 minutes. The activation level of NF-κB (pNF-κB) was analyzed using FACS with anti-human phospho-NF-κB p65 (Ser529) eFlour 660.

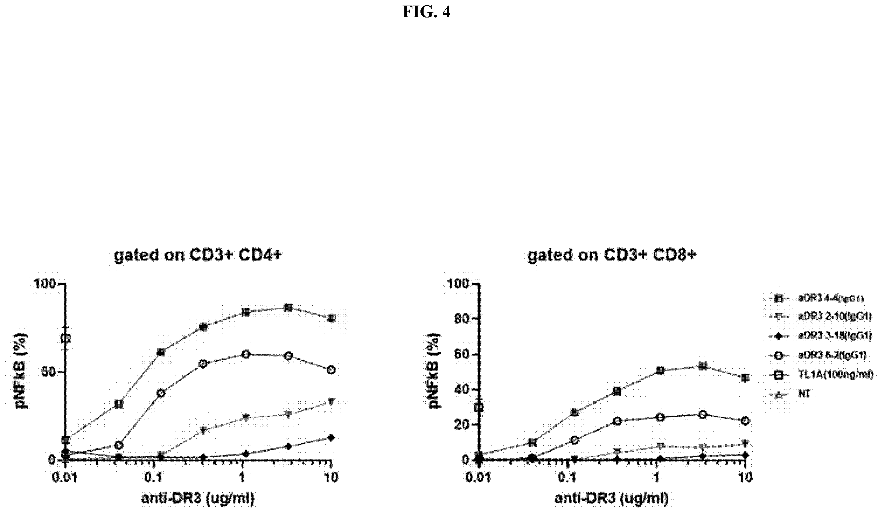

As a result, it was confirmed that the level of NF-κB activation by 4-4 (IgG1) was highest, followed by 6-2. This was found to be because the site where the antibody binds to DR3 overlaps with TL1A and strongly phosphorylates NF-κB (FIG. 4).

### Example 5. Comparison of binding strength according to IgG subclass of Clone 4-4

The functionally best subclass of the anti-DR3 4-4 antibody identified as the best in Example 2 was analyzed. For this purpose, as anti-DR3 4-4 antibodies, clone 4-4 IgG1, clone 4-4 IgG2, and clone 4-4 IgG4 monoclonal antibodies were prepared according to IgG subclass, and ELISA was performed to compare the binding affinity to DR3. Specifically, 0.4 µg/ml DR3-His tag was put into and mixed with PBS, and then a Ni plate was coated with a total of 50 µl of the mixed solution. Thereafter, to prevent non-specific binding, the Ni plate was mixed with 200 µl of PBS consisting of 5% FBS and incubated at room temperature for 30 minutes, and incubated for 30 minutes by titrating the monoclonal antibody. The degree of binding between the coated DR3 and the monoclonal antibody was detected with anti-human IgG Fc HRP. The plate was washed five times with 250 µl of 0.1% Tween 20 at each step.

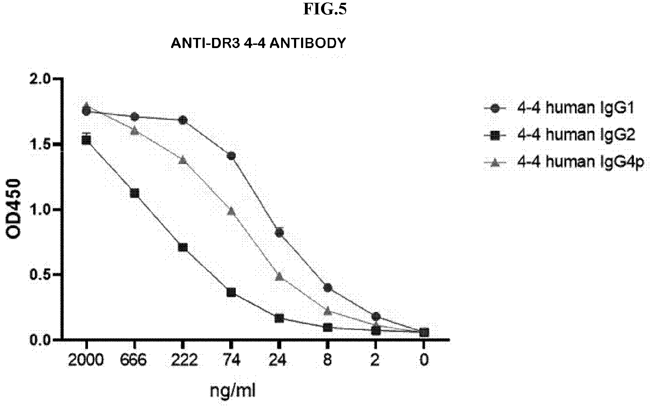

As a result, the anti-DR3 4-4 antibody was confirmed to have excellent affinity for DR3 in the order of subclasses: IgG1, IgG4, and IgG2 (FIG 5).

### Example 6. Confirmation of excellent NF-κB activity effect according to IgG subclass of Clone 4-4

To compare the levels of NF-κB activation according to the IgG subclasses of clone 4-4, FACS was performed after stimulation with clone 4-4 IgG1, clone 4-4 IgG2, and clone 4-4 IgG4 monoclonal antibodies. Specifically, 1×10⁶ hPBMCs were mixed with rhTL1A-His tag or titrated monoclonal antibody, and then incubated at 37 °C for 10 minutes. The level of NF-κB activation was analyzed using FACS with anti-human phospho-NF-κB p65 (Ser529) eFlour 660.

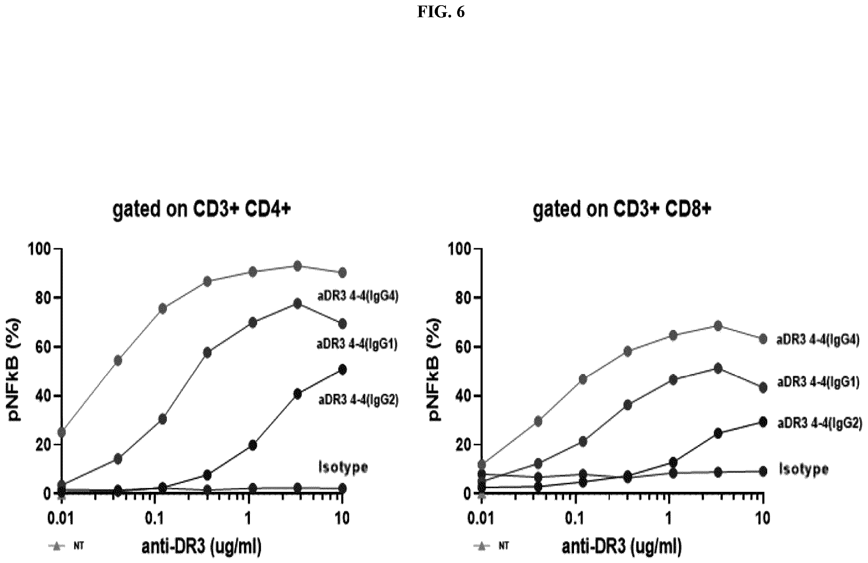

As a result, antibody 4-4 was confirmed to have a high level of NF-κB activation in the order of subclasses: IgG4, IgG1, and IgG2 (FIG 6).

### Example 7. Effect of anti-DR3 4-4 IgG4 antibody on increasing survival rate of regulatory T cells in absence of TCR signal stimulation

In order to confirm whether the anti-DR3 4-4 IgG4 antibody, which was confirmed to be the best in Example 6, can increase the survival rate of human regulatory T cells in the absence of TCR signal stimulation, CD4 T cell culture was performed using anti-DR3 4-4 IgG4 antibody. Specifically, after CD4 T cells were isolated from hPBMC using a Human CD4 T cell Enrichment Kit, the cells were cultured with 20 ng/ml IL-2, 15 ng/ml TL1A, or 1 µg/ml anti-DR34-4 IgG4 antibody for 3 days, and then analyzed by FACS.

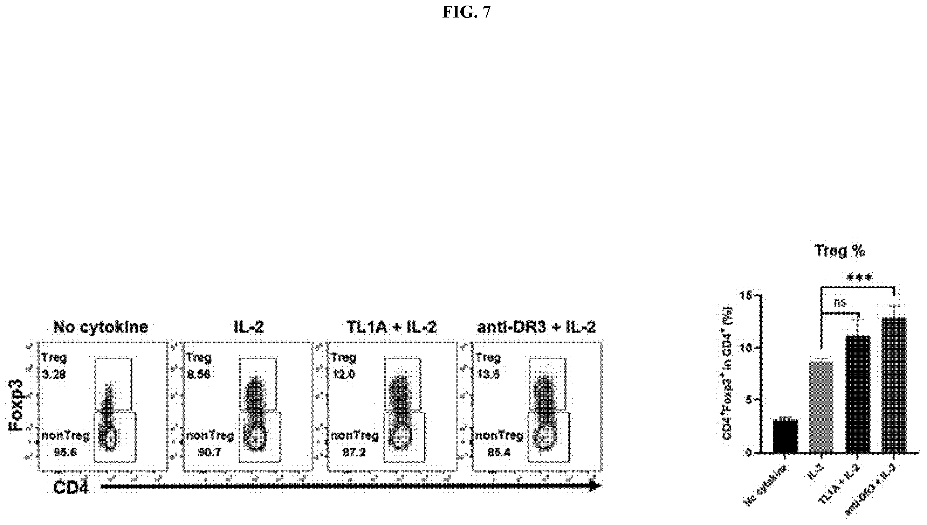

As a result, it was confirmed that in an environment in the absence of TCR signal stimulation, anti-DR3 4-4 IgG4 antibody can increase the survival rate of regulatory T cells to a level similar to that of TL1A, a DR3 ligand (FIG 7).

### Example 8. Effect of anti-DR3 4-4 IgG4 antibody on increasing division and

### survival rate of regulatory T cells according to TCR signal intensity

In order to confirm the degree of human regulatory T cell division promotion of the anti-DR3 4-4 IgG4 antibody, which was confirmed to be the best in Example 6, according to the TCR signal stimulation intensity, CD4 T cell culture was performed using anti-hCD3 antibody and anti-DR3 4-4 IgG4 antibody. Specifically, one day before cell culture, a 96-well cell culture plate was coated with each of 1.5 µg/ml, 0.5 µg/ml, 0.25 µg/ml, 0.125 µg/ml, and 0 µg/ml anti-hCD3ε antibodies, and then on the day of cell culture, CD4 T cells were isolated from hPBMC using the Human CD4 T Cell Enrichment Kit. Thereafter, 0.1×10⁶ CD4 T cells were cultured per well, cultured for 6 days by adding 20 ng/ml IL-2, 0.5 ng/ml TGF-β, or anti-DR3 4-4 IgG4 antibody according to the group, and then analyzed by FACS.

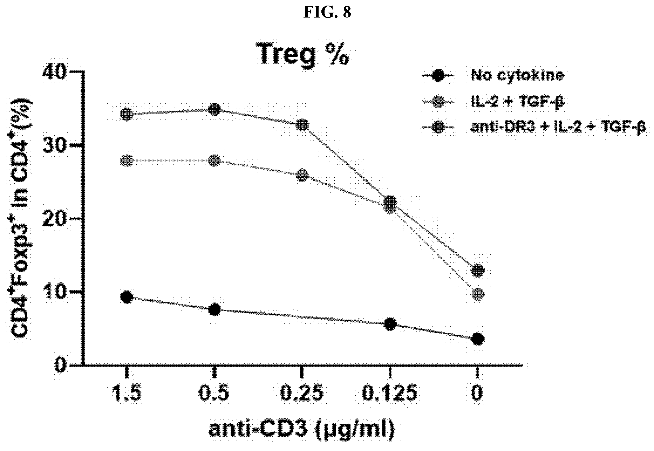

As a result, it was confirmed that anti-DR3 4-4 IgG4 antibody effectively increased the division or survival rate of regulatory T cells when TCR signal stimulation of a certain intensity or higher was given (FIG 8).

### Example 9. Sequencing of anti-DR3 4-4 IgG4 monoclonal antibody

The anti-DR3 4-4 IgG4 monoclonal antibody identified as functionally the best in Examples 3 to 8 was sequenced for application to the treatment of graft-versus-host disease, autoimmune diseases, and type 2 diabetes. Specifically, based on sequence analysis data, the Fab region of 4-4 mAb was cloned into an IgG4 expression vector, and the DNA and amino acid sequences of the cloned vector are shown in Table 4. Table 4 shows the DNA sequence and amino acid sequence of fully human 4-4.

**[Table 4]**

| **SEQ ID NO:** | **Name** | **Sequence** |
|---|---|---|
| SEQ ID NO: 81 | human 4-4 IgG4P mAb DNA sequence heavy chain | GAGGTGCAGCTGGTGGAGAGCGGGGGGGGACTGGTGAAGCCAGGGGGAAGCCTGAGACTGAGCT GCCGCAAGCGGGTTCACATTTAGTGACTATTATATGAGTTGGATCAGACAGGCACCCGGCAAAG CTGGAGTGGGTGAGCTACATCAGCAGCAGTAGCAGCTACACCAACTACGCCGACAGTGTGAAGG AGATTCACCATCAGCAGAGACAACGCCAAGAACAGCCTGTACCTGCAGATGAACAGCCTGAGAG GAGGACACCGCCGTGTACTACTGCGCAAGCAACGTGGACACCGCTATGGTGGGGGCCTTCGACAT TGGGGACAAGGAACACTCGTGACCGTGAGCAGCGCTAGCACCAAGGGCCCATCGGTCTTCCCC GCGCCCTGCTCCAGGAGCACCTCCGAGAGCACAGCCGCCCTGGGCTGCCTGGTCAAGGACTACT CCCGAACCGGTGACGGTGTCGTGGAACTCAGGCGCCCTGACCAGCGGCGTGCACACCTTCCCGG GTCCTACAGTCCTCAGGACTCTACTCCCTCAGCAGCGTGGTGACCGTGCCCTCCAGCAGCTTGG ACGAAGACCTACACCTGCAACGTAGATCACAAGCCCAGCAACACCAAGGTGGACAAGAGAGTTG TCCAAATATGGTCCCCCATGCCCACCATGCCCAGCACCTGAGTTCCTGGGGGGACCATCAGTCT CTGTTCCCCCCAAAACCCAAGGACACTCTCATGATCTCCCGGACCCCTGAGGTCACGTGCGTGG GTGGACGTGAGCCAGGAAGACCCCGAGGTCCAGTTCAACTGGTACGTGGATGGCGTGGAGGTGC AATGCCAAGACAAAGCCGCGGGAGGAGCAGTTCAACAGCACGTACCGTGTGGTCAGCGTCCTCA GTCCTGCACCAGGACTGGCTGAACGGCAAGGAGTACAAGTGCAAGGTCTCCAACAAAGGCCTCC TCCTCCATCGAGAAAACCATCTCCAAAGCCAAAGGGCAGCCCCGAGAGCCACAGGTGTACACCC CCCCCATCCCAGGAGGAGATGACCAAGAACCAGGTCAGCCTGACCTGCCTGGTCAAAGGCTTCT CCCAGCGACATCGCCGTGGAGTGGGAGAGCAATGGGCAGCCGGAGAACAACTACAAGACCACGC CCCGTGCTGGACTCCGACGGCTCCTTCTTCCTCTACAGCAGGCTAACCGTGGACAAGAGCAGGT CAGGAGGGGAATGTCTTCTCATGCTCCGTGATGCATGAGGCTCTGCACAACCACTACACACAGA AGCCTCTCCCTGTCTCTGGGTAAATGA |
| SEQ ID NO: 82 | human 4-4 IgG4P mAb DNA sequence light chain | GACATTGAAATGACCCAGAGCCCCAGCAGCGTGAGCGCCAGCGTGGGAGACACCGTGACCATCA TGCAGAGCAAGCCAACACATAAGCAACTGGCTGGCCTGGTACCAGCAGAAACCCGGACAGGCCCCC AAATTCCTCATCTACACCGCAAGTAACCTGCAAAGCGGCGTGCCCAGCAGATTCAGCGGCAGCG TACGGCACCGACTTCACCCTCACCATCAGCGGACTGCAACCCGAGGACTTCGCCACCTACTACT CAACAAGCCGACAGCTTCCCCCTGACCTTCGGCGGAGGCACCAAGGTGGATATCAAGCGTACGG GCTGCACCATCTGTCTTCATCTTCCCGCCATCTGATGAGCAGTTGAAATCTGGAACTGCCTCTG GTGTGCCTGCTGAATAACTTCTATCCCAGAGAGGCCAAAGTACAGTGGAAGGTGGATAACGCCC CAATCGGGTAACTCCCAGGAGAGTGTCACAGAGCAGGACAGCAAGGACAGCACCTACAGCCTCA AGCACCCTGACGCTGAGCAAAGCAGACTACGAGAAACACAAAGTCTACGCCTGCGAAGTCACCC CAGGGCCTGAGTTCGCCCGTCACAAAGAGCTTCAACAGGGGAGAGTGTTGA |
| SEQ ID NO: 83 | human 4-4 IgG4P mAb amino acid sequence heavy chain | EVQLVESGGGLVKPGGSLRLSCAASGFTFSDYYMSWIRQAPGKGLEWVSYISSSSSYTNYADSV RFTISRDNAKNSLYLQMNSLRAEDTAVYYCASNVDTAMVGAFDIWGQGTLVTVSSASTKGPSVF APCSRSTSESTAALGCLVKDYFPEPVTVSWNSGALTSGVHTFPAVLQSSGLYSLSSVVTVPSSS TKTYTCNVDHKPSNTKVDKRVESKYGPPCPPCPAPEFLGGPSVFLFPPKPKDTLMISRTPEVTC VDVSQEDPEVQFNWYVDGVEVHNAKTKPREEQFNSTYRVVSVLTVLHQDWLNGKEYKCKVSNKG SSIEKTISKAKGQPREPQVYTLPPSQEEMTKNQVSLTCLVKGFYPSDIAVEWESNGQPENNYKT PVLDSDGSFFLYSRLTVDKSRWQEGNVFSCSVMHEALHNHYTQKSLSLSLGK |
| SEQ ID NO: 84 | human 4-4 IgG4P mAb amino acid sequence light chain | DIEMTQSPSSVSASVGDTVTITCRASQHISNWLAWYQQKPGQAPKFLIYTASNLQSGVPSRFSG YGTDFTLTISGLQPEDFATYYCQQADSFPLTFGGGTKVDIKRTVAAPSVFIFPPSDEQLKSGTA VCLLNNFYPREAKVQWKVDNALQSGNSQESVTEQDSKDSTYSLSSTLTLSKADYEKHKVYACEV QGLSSPVTKSFNRGEC |

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described examples are illustrative only in all aspects and are not restrictive.

### [Industrial Applicability]

The antibody that specifically binds to human DR3 can specifically bind to an epitope to which the TLA1 ligand of human DR3 binds, and can selectively act on regulatory T cells to improve the division or survival rate of regulatory T cells while minimizing the activity of effector CD4+ T cells and CD8+ T cells, so the antibody can be usefully used as a therapeutic agent for graft-versus-host disease, autoimmune diseases, or type 2 diabetes, and thus is recognized as industrially applicable.

## Claims

1. An anti-DR3 antibody or an antigen-binding fragment thereof, which specifically binds to death receptor 3 (DR3).

2. The anti-DR3 antibody or antigen-binding fragment thereof of claim 1, wherein the antibody or the antigen-binding fragment is any one selected from the group consisting of the following:
i) an antibody or antigen-binding fragment including a heavy chain variable region including a heavy chain CDR1 set forth in an amino acid sequence of SEQ ID NO: 1, a heavy chain CDR2 set forth in an amino acid sequence of SEQ ID NO: 2, and a heavy chain CDR3 set forth in an amino acid sequence of SEQ ID NO: 3; and a light chain variable region including a light chain CDR1 set forth in an amino acid sequence of SEQ ID NO: 4, a light chain CDR2 set forth in an amino acid sequence of SEQ ID NO: 5, and a light chain CDR3 set forth in an amino acid sequence of SEQ ID NO: 6;
ii) an antibody or antigen-binding fragment including a heavy chain variable region including a heavy chain CDR1 set forth in an amino acid sequence of SEQ ID NO: 7, a heavy chain CDR2 set forth in an amino acid sequence of SEQ ID NO: 8, and a heavy chain CDR3 set forth in an amino acid sequence of SEQ ID NO: 9; and a light chain variable region including a light chain CDR1 set forth in an amino acid sequence of SEQ ID NO: 10, a light chain CDR2 set forth in an amino acid sequence of SEQ ID NO: 11, and a light chain CDR3 set forth in an amino acid sequence of SEQ ID NO: 12;
iii) an antibody or antigen-binding fragment including a heavy chain variable region including a heavy chain CDR1 set forth in an amino acid sequence of SEQ ID NO: 13, a heavy chain CDR2 set forth in an amino acid sequence of SEQ ID NO: 14, and a heavy chain CDR3 set forth in an amino acid sequence of SEQ ID NO: 15; and a light chain variable region including a light chain CDR1 set forth in an amino acid sequence of SEQ ID NO: 16, a light chain CDR2 set forth in an amino acid sequence of SEQ ID NO: 17, and a light chain CDR3 set forth in an amino acid sequence of SEQ ID NO: 18;
iv) an antibody or antigen-binding fragment including a heavy chain variable region including a heavy chain CDR1 set forth in an amino acid sequence of SEQ ID NO: 19, a heavy chain CDR2 set forth in an amino acid sequence of SEQ ID NO: 20, and a heavy chain CDR3 set forth in an amino acid sequence of SEQ ID NO: 21; and a light chain variable region including a light chain CDR1 set forth in an amino acid sequence of SEQ ID NO: 22, a light chain CDR2 set forth in an amino acid sequence of SEQ ID NO: 23, and a light chain CDR3 set forth in an amino acid sequence of SEQ ID NO: 24; and
v) an antibody or antigen-binding fragment including a heavy chain variable region including a heavy chain CDR1 set forth in an amino acid sequence of SEQ ID NO: 25, a heavy chain CDR2 set forth in an amino acid sequence of SEQ ID NO: 26, and a heavy chain CDR3 set forth in an amino acid sequence of SEQ ID NO: 27; and a light chain variable region including a light chain CDR1 set forth in an amino acid sequence of SEQ ID NO: 28, a light chain CDR2 set forth in an amino acid sequence of SEQ ID NO: 29, and a light chain CDR3 set forth in an amino acid sequence of SEQ ID NO: 30.

3. The anti-DR3 antibody or antigen-binding fragment thereof of claim 1, wherein the antibody or the antigen-binding fragment is any one selected from the group consisting of the following:
i) an antibody or antigen-binding fragment thereof including a heavy chain variable region set forth in an amino acid sequence of SEQ ID NO: 63 and a light chain variable region set forth in an amino acid sequence of SEQ ID NO: 64;
ii) an antibody or antigen-binding fragment thereof including a heavy chain variable region set forth in an amino acid sequence of SEQ ID NO: 67 and a light chain variable region set forth in an amino acid sequence of SEQ ID NO: 68;
iii) an antibody or antigen-binding fragment thereof including a heavy chain variable region set forth in an amino acid sequence of SEQ ID NO: 71 and a light chain variable region set forth in an amino acid sequence of SEQ ID NO: 72;
iv) an antibody or antigen-binding fragment thereof including a heavy chain variable region set forth in an amino acid sequence of SEQ ID NO: 75 and a light chain variable region set forth in an amino acid sequence of SEQ ID NO: 76; and
v) an antibody or antigen-binding fragment thereof including a heavy chain variable region set forth in an amino acid sequence of SEQ ID NO: 79 and a light chain variable region set forth in an amino acid sequence of SEQ ID NO: 80.

4. The anti-DR3 antibody or antigen-binding fragment thereof of claim 1, wherein the antibody or the antigen-binding fragment thereof comprises a heavy chain set forth in an amino acid sequence of SEQ ID NO: 83 and a light chain set forth in an amino acid sequence of SEQ ID NO: 84.

5. The anti-DR3 antibody or antigen-binding fragment thereof of claim 4, wherein the antibody or the antigen-binding fragment comprises Fab.

6. The anti-DR3 antibody or antigen-binding fragment thereof of claim 1, wherein the antibody or the antigen-binding fragment is IgG1, or IgG4 subclass.

7. The anti-DR3 antibody or antigen-binding fragment thereof of claim 1, wherein the antigen-binding fragment is any one selected from the group consisting of Fv, Fab, F(ab')2, Fab', dsFv, scFv, sc(Fv)2, and a diabody.

8. The anti-DR3 antibody or antigen-binding fragment thereof of claim 2, wherein the iii), iv), and v) antibody or antigen-binding fragment specifically binds to DR3 competitively with TL1A.

9. The anti-DR3 antibody or antigen-binding fragment thereof of claim 1, wherein the antibody or the antigen-binding fragment is any one or more of the following:
selectively activating regulatory T cells or enhancing the survival rate thereof; and
activating NF-κB.

10. A nucleic acid sequence encoding the antibody or antigen-binding fragment of claim 1.

11. A vector comprising the nucleic acid sequence of claim 10.

12. A transgenic organism transformed with the vector of claim 11.

13. A pharmaceutical composition for preventing or treating graft-versus-host disease, autoimmune diseases, or type 2 diabetes, comprising, as an active ingredient, the anti-DR3 antibody or the antigen-binding fragment thereof, which specifically binds to the death receptor 3 (DR3) of claim 1.

14. The pharmaceutical composition of claim 13, wherein the antibody or the antigen-binding fragment thereof comprises a heavy chain set forth in an amino acid sequence of SEQ ID NO: 83 and a light chain set forth in an amino acid sequence of SEQ ID NO: 84.

15. The pharmaceutical composition of claim 13, wherein the autoimmune disease is any one or more selected from the group consisting of an autoimmune disease caused by graft-versus-host disease, hemophagocytic lymphohistiocytosis, systemic lupus erythematosus, Kikuchi disease, adult onset Still's disease, Behcet disease, IgG4-related disease, type 1 diabetes, systemic sclerosis, psoriasis, multiple sclerosis, and Graves hyperthyroidism.

16. A kit for preventing or treating graft-versus-host disease, autoimmune diseases, or type 2 diabetes, comprising the vector of claim 11 or the composition of claim 13, and instructions.

17. A method for preventing or treating graft-versus-host disease, autoimmune diseases, or type 2 diabetes, the method comprising administering a composition comprising an anti-DR3 antibody or an antigen-binding fragment thereof, which binds to death receptor 3 (DR3), as an active ingredient to a subject in need thereof.

18. A use of a composition comprising an anti-DR3 antibody or an antigen-binding fragment thereof, which binds to death receptor 3 (DR3), as an active ingredient for preventing or treating graft-versus-host disease, autoimmune diseases, or type 2 diabetes.

19. A use of a composition comprising an anti-DR3 antibody or an antigen-binding fragment thereof, which binds to death receptor 3 (DR3), an active ingredient for preparing a preparation for preventing or treating graft-versus-host disease, autoimmune diseases, or type 2 diabetes.
